(19) Europäisches Patentamt
European Patent Office
Office européen des brevets

(11) **EP 4 428 155 A1**

(12) **EUROPEAN PATENT APPLICATION**
published in accordance with Art. 153(4) EPC

(43) Date of publication:
**11.09.2024 Bulletin 2024/37**

(21) Application number: **22889412.7**

(22) Date of filing: **04.11.2022**

(51) International Patent Classification (IPC):
*C07K 16/28* $^{(2006.01)}$   *C12N 15/13* $^{(2006.01)}$
*A61K 39/395* $^{(2006.01)}$   *A61P 35/00* $^{(2006.01)}$

(52) Cooperative Patent Classification (CPC):
**A61K 39/395; A61P 35/00; C07K 16/00;
C07K 16/28**

(86) International application number:
**PCT/CN2022/129875**

(87) International publication number:
**WO 2023/078391 (11.05.2023 Gazette 2023/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.11.2021 CN 202111305831**

(71) Applicant: **CHIA TAI TIANQING
PHARMACEUTICAL GROUP CO., LTD.
Lianyungang,
Jiangsu 222062 (CN)**

(72) Inventors:
• **ZHEN, Zipeng
Nanjing, Jiangsu 211100 (CN)**

• **DU, Xiuzhen
Nanjing, Jiangsu 211100 (CN)**
• **ZHANG, Zhengping
Nanjing , Jiangsu 211100 (CN)**
• **ZHAO, Wei
Nanjing, Jiangsu 211100 (CN)**

(74) Representative: **Dehns
St. Bride's House
10 Salisbury Square
London EC4Y 8JD (GB)**

Remarks:
The complete document including Reference
Table(s) and the Sequence Listing(s) can be
downloaded from the EPO website

(54) **ANTIBODY BINDING TO C-MET AND USE THEREOF**

(57) The present invention relates to an antibody binding to c-Met and a use thereof; specifically, provided is a single variable domain, an antibody or an antigen-binding fragment thereof, or a fusion protein that binds to c-Met, as well as nucleic acids that encode said products, vectors comprising the nucleic acids, cells comprising the vectors, and pharmaceutical compositions comprising same; also provided are detection, pharmaceutical and therapeutic uses thereof.

FIG. 4

**Description**

**TECHNICAL FIELD**

The present disclosure relates to the technical field of molecular biology, and in particular to an antibody or an antigen-binding fragment thereof that binds to c-Met and use thereof.

**BACKGROUND**

The c-mesenchymal-epithelial transition factor (c-Met) is one of the receptor tyrosine kinases, which is a heterodimer of approximately 190 kDa formed by linking a 50 kDa extracellular chain (α chain) and a 145 kDa transmembrane chain (β chain). The transmembrane chain (β chain) includes a SEMA domain (sema homology region, SEMA), a PSI domain (plexin semaphorin-integrin, PSI), 4 immunoglobulin-like repeat domains (immunoglobulin-like regions in plexins and transcription factors, IPT), a transmembrane domain, a juxtamembrane domain (JM), a tyrosine kinase domain (TK), and a carboxy-terminal tail region (carboxyl terminal, CT). c-Met is a receptor expressed on the cell surface, wherein the SEMA domain is one of the important elements for ligand binding and is considered as a binding site of the hepatocyte growth factor (HGF) which is a ligand thereof. HGF is synthesized by mesenchymal cells, fibroblasts, and smooth muscle cells, and activates the HGF/c-Met signal through a paracrine mechanism to exert its biological functions. In various tumors, due to the overexpression of c-Met and HGF, and the paracrine and autocrine positive feedback loops formed by HGF and c-Met, the HGF/c-Met signaling pathway is abnormally activated, and the growth, invasion, migration, and angiogenesis of tumor cells are promoted. Abnormal expression of the c-Met gene is present in many cancer types, such as brain cancer, breast cancer, colorectal cancer, gastric cancer, head and neck cancer, lung cancer, and liver cancer. Immunotherapeutic agents such as antibodies that bind to c-Met are able to block the binding of HGF to c-Met. With c-Met being a potential therapeutic target, some c-Met-targeting antibodies have been developed, but still are limited. There is a need for more available options.

In addition to conventional IgG1 of a four-chain structure, IgG antibodies in animals of the family Camelidae also include naturally occurring IgG2 and IgG3 heavy-chain antibodies (HcAbs) free of light chains. The single variable region domains (V$_H$Hs) of heavy-chain antibodies are capable of specifically binding to antigens and have relatively high affinity for antigens. Based on their uniqueness, V$_H$H domains used as part of an antibody or an antigen-binding fragment have more significant advantages over conventional antibody fragments (such as scFvs and Fabs): for example, only a single domain is required to specifically bind to an antigen with high affinity; they can be easily transformed into polyvalent and multispecific formats; V$_H$H domains are highly soluble and do not tend to aggregate; the V$_H$H molecules are small and thus have relatively high tissue permeability; V$_H$Hs do not need to be paired with light chains, and thus there is no light and heavy chain mismatch problem about forming bispecific or multispecific antibodies.

**SUMMARY**

The present disclosure provides a single variable domain, an antibody or an antigen-binding fragment thereof, or a fusion protein that binds to c-Met. The present disclosure also provides a nucleic acid encoding the provided single variable domain, antibody or antigen-binding fragment thereof, or fusion protein, a vector, a cell, a preparation method, a composition, and use.

In one aspect, the present disclosure provides an isolated single variable domain that binds to c-Met, wherein the single variable domain comprises CDR1, CDR2, and CDR3 in the amino acid sequence set forth in SEQ ID NO: 24, 26, 28, 30, 32, or 34.

In one aspect, the present disclosure provides an isolated single variable domain that binds to c-Met, wherein the single variable domain comprises the following complementarity determining regions: (a) CDR1 comprising the amino acid sequence set forth in SEQ ID NO: 5, 8, 11, 14, 17, or 20; (b) CDR2 comprising the amino acid sequence set forth in SEQ ID NO: 6, 9, 12, 15, 18, or 21; and (c) CDR3 comprising the amino acid sequence set forth in SEQ ID NO: 7, 10, 13, 16, 19, or 22.

In one aspect, the present disclosure provides an isolated antibody or an antigen-binding fragment thereof that binds to c-Met, wherein the antibody or the antigen-binding fragment thereof comprises a single variable domain, wherein the single variable domain comprises CDR1, CDR2, and CDR3 in the amino acid sequence set forth in SEQ ID NO: 24, 26, 28, 30, 32, or 34.

In one aspect, the present disclosure provides an isolated antibody or an antigen-binding fragment thereof that binds to c-Met, wherein the antibody or the antigen-binding fragment thereof comprises a single variable domain, wherein the single variable domain comprises the following complementarity determining regions: (a) CDR1 comprising the amino acid sequence set forth in SEQ ID NO: 5, 8, 11, 14, 17, or 20; (b) CDR2 comprising the amino acid sequence set forth in SEQ ID NO: 6, 9, 12, 15, 18, or 21; and (c) CDR3 comprising the amino acid sequence set forth in SEQ ID NO: 7, 10, 13, 16, 19, or 22.

In one aspect, the present disclosure provides an isolated antibody or an antigen-binding fragment thereof that binds to c-Met, wherein the antibody or the antigen-binding fragment thereof comprises a single variable domain, wherein the single variable domain binds to the same epitope as any one of the single variable domains of the present disclosure.

In one aspect, the present disclosure provides an isolated antibody or an antigen-binding fragment thereof that binds to c-Met, wherein the antibody or the antigen-binding fragment thereof comprises a single variable domain, wherein the single variable domain competes for binding to c-Met with any one of the single variable domains of the present disclosure.

In some embodiments, the single variable domain is a $V_HH$, preferably a camelid $V_HH$ or humanized $V_HH$. In some embodiments, the antibody or the antigen-binding fragment thereof is chimeric or humanized. In some embodiments, the single variable domain is derived from animals of the family Camelidae or is humanized.

In some embodiments, the antibody or the antigen-binding fragment thereof comprises an Fc of IgG. In some embodiments, the antibody or the antigen-binding fragment thereof comprises an Fc of human IgG, preferably an Fc of human IgG1, IgG2, IgG3, or IgG4.

In one aspect, the present disclosure provides a fusion protein comprising the antibody or the antigen-binding fragment thereof of the present disclosure.

In one aspect, the present disclosure provides a pharmaceutical composition comprising the antibody or the antigen-binding fragment thereof of the present disclosure, and a pharmaceutically acceptable carrier.

In one aspect, the present disclosure provides an isolated nucleic acid encoding the antibody or the antigen-binding fragment thereof of the present disclosure.

In one aspect, the present disclosure provides a vector comprising the isolated nucleic acid of the present disclosure.

In one aspect, the present disclosure provides a host cell comprising the isolated nucleic acid or the vector of the present disclosure.

In one aspect, the present disclosure provides a method for preparing the antibody or the antigen-binding fragment thereof of the present disclosure, comprising: culturing the host cell of the present disclosure and isolating the antibody or the antigen-binding fragment thereof expressed, wherein the vector is an expression vector.

In another aspect, the present disclosure provides use of the antibody or the antigen-binding fragment thereof of the present disclosure for preparing a medicament for treating a subject having a tumor.

In another aspect, the present disclosure provides use of the fusion protein of the present disclosure for preparing a medicament for treating a subject having a tumor.

In another aspect, the present disclosure provides use of the pharmaceutical composition of the present disclosure for preparing a medicament for treating a subject having a tumor.

In another aspect, the present disclosure provides a method for detecting or measuring c-Met in a sample, comprising contacting the sample with the antibody or the antigen-binding fragment thereof of the present disclosure, and detecting or measuring a binding complex.

In another aspect, the present disclosure provides a method for inhibiting, reducing or blocking c-Met signaling in a cell, comprising administering to the cell an effective amount of the antibody or the antigen-binding fragment thereof, the fusion protein, or the pharmaceutical composition of the present disclosure. In another aspect, the present disclosure provides a method for inhibiting tumor cell growth or killing a tumor cell, comprising administering to the tumor cell an effective amount of the antibody or the antigen-binding fragment thereof, the fusion protein, or the pharmaceutical composition of the present disclosure.

In another aspect, the present disclosure provides a method for treating a subject having a tumor, comprising administering to the subject a therapeutically effective amount of the antibody or the antigen-binding fragment thereof, the fusion protein, or the pharmaceutical composition of the present disclosure.

**BRIEF DESCRIPTION OF THE DRAWINGS**

FIG. 1A shows curves of flow cytometry assays for binding of anti-human c-Met $V_HH$-$F_C$ chimeric antibodies to H1993 cells.

FIG. 1B shows curves of flow cytometry assays for binding of anti-human c-Met $V_HH$-$F_C$ chimeric antibodies to MKN45 cells.

FIG. 1C shows curves of flow cytometry assays for binding of anti-human c-Met $V_HH$-$F_C$ chimeric antibodies to KP4 cells.

FIG. 1D shows curves of flow cytometry assays for binding of anti-human c-Met $V_HH$-Fc chimeric antibodies to 4MBr-5 cells.

FIG. 2A shows results of competitive ELISA assays for the blocking effect of anti-human c-Met $V_HH$-Fc chimeric antibodies on the binding of HGF to c-Met.

FIG. 2B shows results of competitive ELISA assays for the blocking effect of anti-human c-Met $V_HH$-Fc chimeric antibodies on the binding of HGFβ to c-Met.

FIG. 3 shows assay results of the inhibition effect of anti-human c-Met $V_HH$-Fc chimeric antibodies on c-Met phosphorylation stimulated by HGF in H441 cells.

FIG. 4 shows results of CCK8 assay for the inhibition of KP4 cell proliferation by anti-human c-Met $V_HH$-Fc chimeric antibodies.

## DETAILED DESCRIPTION

### Definitions

Unless otherwise stated, the following terms used in the present disclosure shall have the following meanings. A certain term, unless otherwise specifically defined, should not be considered ambiguous or unclear, but construed according to its common meaning in the art.

The term "antibody" is used in its broadest sense and encompasses a variety of antibody structures including, but not limited to, monoclonal antibodies, polyclonal antibodies, and multispecific antibodies (e.g., bispecific antibodies or trispecific antibodies), so long as they exhibit the desired antigen-binding activity.

The term "antigen-binding fragment" refers to a fragment that retains the antigen-binding function of a full-length antibody, including Fab, Fab', F(ab')$_2$, scFv, Fv, Fd, Fd', a single variable domain (e.g., $V_HH$), and other antibody fragments known in the art, or a modified fragment by any modification known in the art on the basis of the above fragments.

The term "variable domain" or "variable region" refers to a domain of an antibody that is involved in the binding of the antibody to an antigen. Each variable domain of a natural antibody consists essentially of four framework regions and three complementarity determining regions. The four "framework regions" are referred to in the art and herein as "framework region 1" or "FR1", "framework region 2" or "FR2", "framework region 3" or "FR3", and "framework region 4" or "FR4". The framework regions are separated by three complementarity determining regions CDRs referred to in the art and herein as "complementarity determining region 1" or "CDR1", "complementarity determining region 2" or "CDR2", and "complementarity determining region 3" or "CDR3". Thus, the general structure or sequence of a variable domain can be represented as follows: FR1-CDR1-FR2-CDR2-FR3-CDR3-FR4. Variable domains impart specificity for an antigen to an antibody by virtue of having an antigen-binding site.

The term "single variable domain" refers to having a variable domain capable of specifically binding to an antigen epitope without being paired with other variable domains. The antigen-binding site of a single variable domain is typically formed from three CDRs (CDR1, CDR2 and CDR3) and present on a single domain. In some cases, a single variable domain may be a heavy chain variable domain (e.g., $V_H$) or a suitable fragment thereof, so long as it can form a single antigen-binding unit (i.e., a functional antigen-binding unit consisting essentially of a single variable domain; in this way, a single antigen-binding domain can form a functional antigen-binding unit without interacting with another variable domain). Another example of a single variable domain is "$V_HH$ domain" of the family Camelidae, also known as VHH domain, $V_HH$, or VHH.

The term "$V_HH$ domain" is used to distinguish these variable domains from the heavy chain variable domains present in conventional four-chain antibodies (which are referred to herein as "$V_H$ domains" or "$V_Hs$") and the light chain variable domains present in conventional four-chain antibodies (which are referred to herein as "$V_L$ domains" or "$V_Ls$"). $V_HH$ domains specifically bind to epitopes without additional antigen-binding domains involved (it is different with the $V_H$ or $V_L$ domains of conventional four-chain antibodies, and in this case epitopes are recognized by both the $V_L$ and $V_H$ domains). $V_HH$ domains are small, stable and efficient antigen recognition units formed from a single domain.

The term "framework region" (FR) residues refers to amino acid residues of variable domains other than the CDR residues defined herein.

"complementarity determining region" (CDR) is also referred to as "hypervariable region (HVR)". Natural four-chain antibodies typically comprise six CDRs, three in the heavy chain variable region (heavy chain CDR1, heavy chain CDR2, and heavy chain CDR3) and three in the light chain variable region (light chain CDR1, light chain CDR2, and light chain CDR3). A heavy-chain antibody or a single variable domain typically has three CDRs (CDR1, CDR2, and CDR3). CDR3 is the most diverse of the three CDRs and is believed to play a unique role in imparting fine specificity to antibodies.

There are currently many ways to define CDRs. The Kabat scheme defines CDRs based on the sequence variability and is the most commonly used (Elvin A. Kabat, et al., Sequences of Proteins of Immunological Interest, 5th Edition, Public Health Service, National Institutes of Health, Bethesda, Md. (1991)), while the Chothia scheme defines CDRs based on the positions of structural loops (Cyrus Chothia, et al., Canonical Structures for the Hypervariable Regions of Immunoglobulins, J. Mol. Biol. 196:901-917(1987)). The AbM scheme, a compromise between the Kabat scheme and the Chothia scheme, is used by the Oxford Molecular's AbM antibody modeling software. "Contact" defines CDRs based on the analysis of the crystal structure of available complexes. However, it should be noted that boundaries of the CDRs of variable regions of the same antibody based on different methods and definitions may differ, that is, CDR sequences of the variable regions of the same antibody defined by different methods may differ. Thus, when reference is made to an antibody defined with a particular CDR sequence defined herein, the scope of the antibody also encompasses antibodies defined by CDR sequences that are converted to other arbitrary definitions (e.g., a combination of one or more of the IMGT, Chothia, AbM definitions, etc.).

The term "human consensus framework" or "acceptor human framework" refers to a framework of the most frequently occurring amino acid residues in selecting human immunoglobulin $V_L$ or $V_H$ framework sequences. In general, human immunoglobulin $V_L$ or $V_H$ sequences are selected from a subgroup of variable domain sequences. In general, the subgroup of sequences is that in Kabat et al, Sequences of Proteins of Immunological Interest, 5th Edition, Public Health Service, National Institutes of Health, Bethesda, Md. (1991). Examples include: for $V_L$, the subgroup may be subgroup κI, κII, κIII or κIV described by Kabat et al. (from the same source as is described above). In addition, for $V_H$, the subgroup may be subgroup I, subgroup II or subgroup III described by Kabat et al. Alternatively, the human consensus framework region may be derived from particular residues described above; for example, when human framework residues are selected according to the homology of human framework region residues to a donor framework region by aligning the donor framework region sequence to a set of various human framework region sequences, the acceptor human framework "derived from" the human consensus framework region may comprise the same amino acid sequence, or it may comprise pre-existing amino acid sequence variations. In some embodiments, the number of pre-existing amino acid changes is 10 or less, 9 or less, 8 or less, 7 or less, 6 or less, 5 or less, 4 or less, 3 or less, or 2 or less.

The term "Fc domain" or "Fc" is used to define the C-terminal region of an immunoglobulin heavy chain, which comprises at least part of a constant region. The term includes natural sequence Fc and variant Fc. The C-terminal lysine of Fc (Lys447 according to the EU numbering system) may be present or not.

The term $EC_{50}$ refers to the effective concentration, 50% of the maximal response of a molecule of a certain compound (e.g., an antibody or an antigen-binding fragment thereof). The term "$IC_{50}$" refers to the inhibitory concentration, 50% of the maximal response of a molecule of a certain compound (e.g., an antibody or an antigen-binding fragment thereof). Both $EC_{50}$ and $IC_{50}$ may be measured by ELISA or FACS assay or any other method known in the art.

The term "$K_a$" or "association rate constant" refers to the association rate constant of a molecule of a certain compound (e.g., an antibody or an antigen-binding fragment thereof) that binds to an antigen to form a complex. The term "$K_d$" or "dissociation rate constant" refers to the dissociation rate constant of the dissociation of a molecule of a certain compound (e.g., an antibody or an antigen-binding fragment thereof) from a complex. The term "$K_D$" or "equilibrium dissociation constant" refers to the value obtained at equilibrium in a titration measurement, or by dividing the dissociation rate constant ($K_d$) by the association rate constant ($K_a$). The binding affinity between a molecule of a certain compound and an antigen is expressed using $K_a$, $K_d$, and $K_D$. Methods for determining values of $K_a$, $K_d$, and KD are well known in the art.

The term "pharmaceutically acceptable" refers to a substance, such as a carrier or diluent, that does not eliminate the biological activity or properties of the compounds described herein. Such substances are administered to an individual without causing undesirable biological effects or interacting in a deleterious manner with any of the components of the composition in which it is contained.

The term "pharmaceutically acceptable carrier" includes any and all solvents, dispersion media, coating materials, surfactants, antioxidants, preservatives (e.g., antibacterial agents and antifungal agents), isotonizing agents, absorption delaying agents, salts, preservatives, drug stabilizers, binders, excipients, disintegrants, lubricants, sweeteners, flavoring agents, dyes, and the like, and combinations thereof, as are well known to those skilled in the art (Remington's Pharmaceutical Sciences, 18th Ed. Mack Printing Company, 1990, pp. 1289-1329). The use of any conventional carrier in therapeutic or pharmaceutical compositions is contemplated unless it is incompatible with the active ingredient.

The term "treating" or "treatment" refers to an attempt to alter the natural course of a disease in a treated individual, and may be a clinical intervention performed for prophylaxis or during the course of clinical pathology. Desired therapeutic effects include, but are not limited to, preventing the occurrence or recurrence of diseases, relieving symptoms, reducing any direct or indirect pathological outcomes of diseases, preventing metastasis, slowing disease progression rates, improving or alleviating disease conditions, and regressing or improving prognosis.

As used herein, the term "therapeutically effective amount" refers to the amount of a compound, a pharmaceutical composition, or a pharmaceutical combination necessary to provide a therapeutic benefit to a subject.

The term "subject" includes any human or non-human animal. The term "non-human animal" includes all vertebrates, e.g., mammals and non-mammals, such as non-human primates, sheep, dogs, cats, horses, cows, chicken, amphibians, and reptiles. Preferably, the subject according to the present disclosure is a human. The terms "patient" and "subject" are used interchangeably unless otherwise indicated.

The term "specific binding" or "specifically bind to" means that the binding is selective for an antigen and can be distinguished from interactions that are not desired or are non-specific. The binding ability of a molecule of a certain compound (e.g., an antibody or an antigen-binding fragment thereof) to a particular antigenic determinant may be measured by enzyme-linked immunosorbent assay (ELISA) or other techniques familiar to those skilled in the art, e.g., the surface plasmon resonance (SPR) technique (analysis on a Biacore instrument).

The term "isolated" means that a target compound, such as an antibody or an antigen-binding fragment thereof, $V_HH$, or a nucleic acid, has been isolated from its natural environment.

The term sequence "identity" is also known as consistency. The percent identity between two sequences is a function of identical positions shared by the sequences (i.e., %identity = number of identical positions/total number of positions $\times$ 100), where the number of gaps and the length of each gap that need to be introduced to produce an optimal alignment of the two sequences should be taken into consideration. As shown in the following non-limiting examples, comparison of sequences and determination of percent identity between two sequences can be accomplished using a mathematical algorithm. The percent identity between two amino acid sequences can be determined using the algorithm of E. Meyers and W Miller (Comput. Appl. Biosci., 4:11-17 (1988)), which has been incorporated into the ALIGN program (version 2.0) and uses a PAM120 residue weight table with a gap length penalty of 12 and a gap penalty of 4. In addition, the percent identity of two amino acid sequences can be determined using the algorithm of Needleman and Wunsch (J. Mol. Biol., 484 453 (1970)), which has been incorporated into the GAP program in the GCG software package (available at www.gcg.com) and uses the Blossum 62 matrix or the PAM250 matrix with a gap weight of 16, 14, 12, 10, 8, 6 or 4 and a length weight of 1, 2, 3, 4, 5 or 6.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, about may mean a range of up to 20% (i.e., $\pm$20%), for example, fluctuating within a particular numerical range given $\pm$10%, $\pm$5%, $\pm$2%, $\pm$1% or $\pm$0.5%. Furthermore, particularly with respect to biological systems or methods, the term may refer to being up to an order of magnitude or up to 5 times a certain value. Where a particular value is given in the present disclosure or in the claims, unless otherwise stated, "about" should be considered to mean being within an acceptable error range for that particular value.

Amino acid residues in proteins are abbreviated as follows: phenylalanine is Phe or F; leucine is Leu or L; isoleucine is Ile or I; methionine is Met or M; valine is Val or V; serine is Ser or S; proline is Pro or P; threonine is Thr or T; alanine is Ala or A; tyrosine is Tyr or Y; histidine is His or H; glutamine is Gln or Q; asparagine is Asn or N; lysine is Lys or K; aspartic acid is Asp or D; glutamic acid is Glu or E; cysteine is Cys or C; tryptophan is Trp or W; arginine is Arg or R; glycine is Gly or G.

Various aspects of the present disclosure will be described in further detail in the following sections.

*Single variable domain*

The present disclosure provides an isolated single variable domain that binds to c-Met (such as human c-Met). The single variable domain provides more available options for the development or drug construction of c-Met-targeting drugs. The single variable domain has a number of desirable therapeutic properties such as one or more of good affinity for human c-Met, capability of blocking the binding of ligand HGF or a β chain thereof to c-Met, and inhibition of c-Met phosphorylation caused by HGF stimulation.

In some embodiments, the single variable domain binds to c-Met, which comprises one, two or all three CDRs of the variable region sequence set forth in SEQ ID NO: 24. In one specific embodiment, the single variable domain binds to c-Met, which comprises CDR1, CDR2, and CDR3 of the variable region sequence set forth in SEQ ID NO: 24. In some embodiments, the single variable domain binds to c-Met, which comprises one, two or all three CDRs of the variable region sequence set forth in SEQ ID NO: 26. In one specific embodiment, the single variable domain binds to c-Met, which comprises CDR1, CDR2, and CDR3 of the variable region sequence set forth in SEQ ID NO: 26. In some embodiments, the single variable domain binds to c-Met, which comprises one, two or all three CDRs of the variable region sequence set forth in SEQ ID NO: 28. In one specific embodiment, the single variable domain binds to c-Met, which comprises CDR1, CDR2, and CDR3 of the variable region sequence set forth in SEQ ID NO: 28. In some embodiments, the single variable domain binds to c-Met, which comprises one, two or all three CDRs of the variable region sequence set forth in SEQ ID NO: 30. In one specific embodiment, the single variable domain binds to c-Met, which comprises CDR1, CDR2, and CDR3 of the variable region sequence set forth in SEQ ID NO: 30. In some embodiments, the single variable domain binds to c-Met, which comprises one, two or all three CDRs of the variable region sequence set forth in SEQ ID NO: 32. In one specific embodiment, the single variable domain binds to c-Met, which comprises CDR1, CDR2, and CDR3 of the variable region sequence set forth in SEQ ID NO: 32. In some embodiments, the single variable domain binds to c-Met, which comprises one, two or all three CDRs of the variable region sequence set forth in SEQ ID NO: 34. In one specific embodiment, the single variable domain binds to c-Met, which comprises CDR1, CDR2, and CDR3 of the variable region sequence set forth in SEQ ID NO: 34. In some embodiments, the single variable domain is derived from animals of the family Camelidae. In some embodiments, the single variable domain is humanized.

In some embodiments, the single variable domain comprises an acceptor human framework.

In some embodiments, the single variable domain binds to c-Met, which comprises at least one, at least two, or all three CDRs selected from the following: (a) CDR1 comprising the amino acid sequence set forth in SEQ ID NO: 5, 8, 11, 14, 17, or 20; (b) CDR2 comprising the amino acid sequence set forth in SEQ ID NO: 6, 9, 12, 15, 18, or 21; and (c) CDR3 comprising the amino acid sequence set forth in SEQ ID NO: 7, 10, 13, 16, 19, or 22. In some embodiments, the single variable domain binds to c-Met, which comprises the following complementarity determining regions: (a) CDR1 comprising the amino acid sequence set forth in SEQ ID NO: 5, 8, 11, 14, 17, or 20; (b) CDR2 comprising the amino acid sequence set forth in SEQ ID NO: 6, 9, 12, 15, 18, or 21; and (c) CDR3 comprising the amino acid sequence set forth in SEQ ID NO: 7, 10, 13, 16, 19, or 22. In some embodiments, the single variable domain is derived from animals of the family Camelidae. In some embodiments, the single variable domain is humanized. In some embodiments, the single variable domain comprises an acceptor human framework.

In some embodiments, the single variable domain binds to c-Met, which comprises CDR1 comprising the amino acid sequence set forth in SEQ ID NO: 5, CDR2 comprising the amino acid sequence set forth in SEQ ID NO: 6, and CDR3 comprising the amino acid sequence set forth in SEQ ID NO: 7. In some embodiments, the single variable domain binds to c-Met, which comprises CDR1 comprising the amino acid sequence set forth in SEQ ID NO: 8, CDR2 comprising the amino acid sequence set forth in SEQ ID NO: 9, and CDR3 comprising the amino acid sequence set forth in SEQ ID NO: 10. In some embodiments, the single variable domain binds to c-Met, which comprises CDR1 comprising the amino acid sequence set forth in SEQ ID NO: 11, CDR2 comprising the amino acid sequence set forth in SEQ ID NO: 12, and CDR3 comprising the amino acid sequence set forth in SEQ ID NO: 13. In some embodiments, the single variable domain binds to c-Met, which comprises CDR1 comprising the amino acid sequence set forth in SEQ ID NO: 14, CDR2 comprising the amino acid sequence set forth in SEQ ID NO: 15, and CDR3 comprising the amino acid sequence set forth in SEQ ID NO: 16. In some embodiments, the single variable domain binds to c-Met, which comprises CDR1 comprising the amino acid sequence set forth in SEQ ID NO: 17, CDR2 comprising the amino acid sequence set forth in SEQ ID NO: 18, and CDR3 comprising the amino acid sequence set forth in SEQ ID NO: 19. In some embodiments, the single variable domain binds to c-Met, which comprises CDR1 comprising the amino acid sequence set forth in SEQ ID NO: 20, CDR2 comprising the amino acid sequence set forth in SEQ ID NO: 21, and CDR3 comprising the amino acid sequence set forth in SEQ ID NO: 22. In some embodiments, the single variable domain is derived from animals of the family Camelidae. In some embodiments, the single variable domain is humanized. In some embodiments, the single variable domain comprises an acceptor human framework.

In some embodiments, the single variable domain binds to c-Met, which comprises CDR1 of the amino acid sequence set forth in SEQ ID NO: 5, CDR2 of the amino acid sequence set forth in SEQ ID NO: 6, and CDR3 of the amino acid sequence set forth in SEQ ID NO: 7. In some embodiments, the single variable domain binds to c-Met, which comprises CDR1 of the amino acid sequence set forth in SEQ ID NO: 8, CDR2 of the amino acid sequence set forth in SEQ ID NO: 9, and CDR3 of the amino acid sequence set forth in SEQ ID NO: 10. In some embodiments, the single variable domain binds to c-Met, which comprises CDR1 of the amino acid sequence set forth in SEQ ID NO: 11, CDR2 of the amino acid sequence set forth in SEQ ID NO: 12, and CDR3 of the amino acid sequence set forth in SEQ ID NO: 13. In some embodiments, the single variable domain binds to c-Met, which comprises CDR1 of the amino acid sequence set forth in SEQ ID NO: 14, CDR2 of the amino acid sequence set forth in SEQ ID NO: 15, and CDR3 of the amino acid sequence set forth in SEQ ID NO: 16. In some embodiments, the single variable domain binds to c-Met, which comprises CDR1 of the amino acid sequence set forth in SEQ ID NO: 17, CDR2 of the amino acid sequence set forth in SEQ ID NO: 18, and CDR3 of the amino acid sequence set forth in SEQ ID NO: 19. In some embodiments, the single variable domain binds to c-Met, which comprises CDR1 of the amino acid sequence set forth in SEQ ID NO: 20, CDR2 of the amino acid sequence set forth in SEQ ID NO: 21, and CDR3 of the amino acid sequence set forth in SEQ ID NO: 22. In some embodiments, the single variable domain is derived from animals of the family Camelidae. In some embodiments, the single variable domain is humanized. In some embodiments, the single variable domain comprises an acceptor human framework.

In some embodiments, the single variable domain binds to c-Met, which comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24, 26, 28, 30, 32, or 34; optionally, the single variable domain comprises the particular CDR1, CDR2, and/or CDR3 sequences described above.

In some embodiments, the single variable domain binds to c-Met, which comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24, and the single variable domain comprises: CDR1 comprising the amino acid sequence set forth in SEQ ID NO: 5, CDR2 comprising the amino acid sequence set forth in SEQ ID NO: 6, and CDR3 comprising the amino acid sequence set forth in SEQ ID NO: 7.

In some embodiments, the single variable domain binds to c-Met, which comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 26, and the single variable domain comprises: CDR1 comprising the amino acid sequence set forth in SEQ ID NO: 8, CDR2 comprising the amino acid sequence set forth in SEQ ID NO: 9, and CDR3 comprising the amino acid sequence set forth in SEQ ID NO: 10.

In some embodiments, the single variable domain binds to c-Met, which comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 28, and the single variable domain comprises: CDR1 comprising the amino acid sequence set forth in SEQ ID NO: 11, CDR2 comprising the amino acid sequence set forth in SEQ ID NO: 12, and CDR3 comprising the amino acid sequence set forth in SEQ ID NO: 13.

In some embodiments, the single variable domain binds to c-Met, which comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 30, and the single variable domain comprises: CDR1 comprising the amino acid sequence set forth in SEQ ID NO: 14, CDR2 comprising the amino acid sequence set forth in SEQ ID NO: 15, and CDR3 comprising the amino acid sequence set forth in SEQ ID NO: 16.

In some embodiments, the single variable domain binds to c-Met, which comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32, and the single variable domain comprises: CDR1 comprising the amino acid sequence set forth in SEQ ID NO: 17, CDR2 comprising the amino acid sequence set forth in SEQ ID NO: 18, and CDR3 comprising the amino acid sequence set forth in SEQ ID NO: 19.

In some embodiments, the single variable domain binds to c-Met, which comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 34, and the single variable domain comprises: CDR1 comprising the amino acid sequence set forth in SEQ ID NO: 20, CDR2 comprising the amino acid sequence set forth in SEQ ID NO: 21, and CDR3 comprising the amino acid sequence set forth in SEQ ID NO: 22.

In some embodiments, a single variable domain amino acid sequence having at least 85%, 86%, 87%, 88%, 89%, 90%, 91%, 92%, 93%, 94%, 95%, 96%, 97%, 98%, 99%, or 100% identity comprises substitutions (e.g., conservative substitutions), insertions, or deletions compared to a reference sequence; however, single variable domains comprising the sequence retain the ability to bind to c-Met. In some embodiments, a total of 1-18, 1-16, 1-14, 1-12, 1-10, 1-9, 1-8, 1-7, 1-6, 1-5, 1-4, 1-3, or 1-2 amino acids are substituted, inserted and/or deleted in the amino acid sequence selected from SEQ ID NOs: 24, 26, 28, 30, 32, and 34. In some embodiments, the substitutions, insertions or deletions occur in regions outside of CDRs (i.e., in FRs). In some embodiments, the substitutions, insertions or deletions occur in CDR regions, for example, in one, two, or three of CDR1, CDR2, and CDR3. In some embodiments, the substitutions, insertions or deletions occur in CDR regions and non-CDR regions. Optionally, the single variable domain comprises the amino acid sequence selected from SEQ ID NOs: 24, 26, 28, 30, 32, and 34, including post-translational modifications of the sequence.

In some embodiments, the single variable domain binds to c-Met, which comprises the amino acid sequence set forth in SEQ ID NO: 24, 26, 28, 30, 32, or 34.

In some embodiments, the single variable domain (including any of the embodiments described above, e.g., the single variable domain described above comprising particular CDR1, CDR2 and/or CDR3) is a V$_H$H. In some specific embodiments, V$_H$H has the following structure from N-terminus to C-terminus: FR1-CDR1-FR2-CDR2-FR3-CDR3-FR4.

In some embodiments, the single variable domain (including any of the embodiments described above, e.g., the single variable domain described above comprising particular CDR1, CDR2 and/or CDR3) is a humanized V$_H$H. A V$_H$H of non-human origin may be "humanized" by replacing one or more amino acid residues in the amino acid sequence of the original V$_H$H with one or more amino acid residues present at corresponding positions in the V$_H$ domain of a human conventional four-chain antibody. Humanization may be desirable to reduce immunogenicity.

In some embodiments, the single variable domain binds to c-Met, and the amino acid sequence thereof is set forth in SEQ ID NO: 24, 26, 28, 30, 32, or 34.

The present disclosure also provides a single variable domain that binds to the same epitope as any one of the single variable domains of the present disclosure. In some specific embodiments, the single variable domain binds to the same epitope as a single variable domain comprising the amino acid sequence set forth in SEQ ID NO: 24, 26, 28, 30, 32, or 34. In some embodiments, the single variable domain that binds to the same epitope is derived from animals of the family Camelidae or is humanized.

Based on the binding to the same epitope, competitive screening of single variable domains can be carried out using conventional techniques known to those skilled in the art. For example, competitive studies can be carried out to obtain single variable domains that compete with each other for binding to antigens. Thus, the present disclosure also provides a single variable domain that competes for binding to c-Met with any one of the single variable domains described in the present disclosure. In some specific embodiments, the single variable domain competes for binding to c-Met with a single variable domain comprising the amino acid sequence set forth in SEQ ID NO: 24, 26, 28, 30, 32, or 34. The binding to c-Met can be measured by ELISA, flow cytometry or surface plasmon resonance (SPR) assay, or any other method known in the art. In some embodiments, the single variable domain that competes for binding to c-Met is derived from animals of the family Camelidae or is humanized.

In some embodiments, the single variable domain according to any one of the embodiments described above may comprise one or several of the following properties and characteristics, alone or in combination:

(i) binding to human c-Met or/and monkey c-Met;
(ii) blocking the binding of HGF or a β chain thereof to c-Met; or
(iii) inhibiting c-Met phosphorylation caused by HGF stimulation.

In some embodiments, the single variable domain binds to c-Met. In some embodiments, the single variable domain blocks the binding of HGF or a β chain thereof to c-Met. In some embodiments, the single variable domain inhibits c-Met phosphorylation caused by HGF stimulation.

The present disclosure provides some exemplary single variable domains that bind to c-Met. The amino acid sequences of the CDRs (CDR1, CDR2 and CDR3) of the exemplary single variable domains provided by the present disclosure are provided in Table S 1 below. The full-length amino acid sequences of the exemplary single variable domains are provided in Table S2 below.

Table S1. CDR sequences of exemplary single variable domains

| Name | CDR1 | CDR2 | CDR3 |
|---|---|---|---|
| 1B-3B11 single variable domain | SVA (SEQ ID NO: 5) | VINPGGDNTDYTYAVKG (SEQ ID NO: 6) | GRTFGGTWMDRNKWKY (SEQ ID NO: 7) |
| 1B-1B6 single variable domain | SGCMG (SEQ ID NO: 8) | TIYTRNDFTYYSDSVKG (SEQ ID NO: 9) | GSPFRYAGAWNVCGLKEEDYNY (SEQ ID NO: 10) |
| 1B-1C7 single variable domain | ANCVG (SEQ ID NO: 11) | AINTGGETATYADFVKG (SEQ ID NO: 12) | VGTRKYWDECSLSQHAYKN (SEQ ID NO: 13) |
| 1B-1A8 single variable domain | SYAMD (SEQ ID NO: 14) | GINSAGSPSYEASVKG (SEQ ID NO: 15) | CRDFRSPYCR (SEQ ID NO: 16) |
| 1B-1B2 single variable domain | MG (SEQ ID NO: 17) | SISSDGNSDYLDSVKG (SEQ ID NO: 18) | NGAGHLVLRPLCGSNAVDA (SEQ ID NO: 19) |
| 3B-1C7 single variable domain | GTTMG (SEQ ID NO: 20) | DISSDGNTYYSDSVKG (SEQ ID NO: 21) | AEFRLLLRPLCRQDDYGY (SEQ ID NO: 22) |

Table S2. Full-length sequences of exemplary single variable domains

| Name | Amino acid sequence | SEQ ID NO: |
|---|---|---|
| 1B-3B11 single variable domain | QVQLVESGGGSVQAGGSLRLSCVVSGYTYSSVAWFRQTPG KEREGVAVINPGGDNTDYTYAVKGRFTISRDSAKSTVFLQM NNLESEDTAMYYCAAGRTFGGTWMDRNKWKYWGQGTL VTVSS | 24 |
| 1B-1B6 single variable domain | QVQLVESGGDSVQAGGSLRLSCAASAHAYSSGCMGWFRQ APGKGREGVATIYTRNDFTYYSDSVKGRFTISRDDAKNTV YLQVNSLKAEDTAMYYCAAGSPFRYAGAWNVCGLKEEDY NYWGQGTLVTVSS | 26 |
| 1B-1C7 single variable domain | QVQLVESGGGLVQPGGSLRLSCAASGYSYSANCVGWFYQ APGKEREGVAAINTGGETATYADFVKGRFTISQDYAKNTVY LQMNSLKSEDTGMYYCAAVGTRKYWDECSLSQHAYKNW GQGTLVTVSS | 28 |

(continued)

| Name | Amino acid sequence | SEQID NO: |
|---|---|---|
| 1B-1A8 single variable domain | EVQLVESGGGLVQPGGSLRLSCVASGFTFKSYAMDWVRQA PGKGLEWIAGINSAGSPSYEASVKGRFTISRDNAENTLYLQ LNSLKTEDTAMYYCTKCRDFRSPYCRPGQGTLVTVSS | 30 |
| 1B-1B2 single variable domain | EVQLVESGGGSVQAGGSLRLSCTASGFTFDMGWYRQAPG DECELVSSISSDGNSDYLDSVKGRFTISQDNAKNTVYLQM NSLKPEDTAVYYCAANGAGHLVLRPLCGSNAVDAWGQGT LVTVSS | 32 |
| 3B-1C7 single variable domain | QVQLVESGGDSVQAGGSLRLSCIASGFTFDGTTMGWYRQ APGDECELVSDISSDGNTYYSDSVKGRFTISQGNAKNTVYL QMNSLTPEDTAVYYCAAAEFRLLLRPLCRQDDYGYWGQG TLVTVSS | 34 |

*Antibody or antigen-binding fragment thereof*

The present disclosure provides an isolated antibody or an antigen-binding fragment thereof that binds to c-Met (such as human c-Met), wherein the antibody or the antigen-binding fragment thereof comprises at least one (one or more) single variable domain of the present disclosure.

The antibody or the antigen-binding fragment thereof that binds to c-Met provided by the present disclosure has a number of desirable therapeutic properties such as one or more of good affinity for human c-Met, capability of blocking the binding of ligand HGF or a β chain thereof to c-Met, inhibition of c-Met phosphorylation caused by HGF stimulation, good binding performance for cells with different c-Met expression levels, and killing of tumor cells.

In some embodiments, the antibody or the antigen-binding fragment thereof binds to c-Met and comprises at least one (one or more) single variable domain, wherein the single variable domain comprises one, two or all three CDRs of the variable region sequence set forth in SEQ ID NO: 24. In one specific embodiment, the antibody or the antigen-binding fragment thereof binds to c-Met and comprises at least one (one or more) single variable domain, wherein the single variable domain comprises CDR1, CDR2, and CDR3 of the variable region sequence set forth in SEQ ID NO: 24. In some embodiments, the antibody or the antigen-binding fragment thereof binds to c-Met and comprises at least one (one or more) single variable domain, wherein the single variable domain comprises one, two or all three CDRs of the variable region sequence set forth in SEQ ID NO: 26. In one specific embodiment, the antibody or the antigen-binding fragment thereof binds to c-Met and comprises at least one (one or more) single variable domain, wherein the single variable domain comprises CDR1, CDR2, and CDR3 of the variable region sequence set forth in SEQ ID NO: 26. In some embodiments, the antibody or the antigen-binding fragment thereof binds to c-Met and comprises at least one (one or more) single variable domain, wherein the single variable domain comprises one, two or all three CDRs of the variable region sequence set forth in SEQ ID NO: 28. In one specific embodiment, the antibody or the antigen-binding fragment thereof binds to c-Met and comprises at least one (one or more) single variable domain, wherein the single variable domain comprises CDR1, CDR2, and CDR3 of the variable region sequence set forth in SEQ ID NO: 28. In some embodiments, the antibody or the antigen-binding fragment thereof binds to c-Met and comprises at least one (one or more) single variable domain, wherein the single variable domain comprises one, two or all three CDRs of the variable region sequence set forth in SEQ ID NO: 30. In one specific embodiment, the antibody or the antigen-binding fragment thereof binds to c-Met and comprises at least one (one or more) single variable domain, wherein the single variable domain comprises CDR1, CDR2, and CDR3 of the variable region sequence set forth in SEQ ID NO: 30. In some embodiments, the antibody or the antigen-binding fragment thereof binds to c-Met and comprises at least one (one or more) single variable domain, wherein the single variable domain comprises one, two or all three CDRs of the variable region sequence set forth in SEQ ID NO: 32. In one specific embodiment, the antibody or the antigen-binding fragment thereof binds to c-Met and comprises at least one (one or more) single variable domain, wherein the single variable domain comprises CDR1, CDR2, and CDR3 of the variable region sequence set forth in SEQ ID NO: 32. In some embodiments, the antibody or the antigen-binding fragment thereof binds to c-Met and comprises at least one (one or more) single variable domain, wherein the single variable domain comprises one, two or all three CDRs of the variable region sequence set forth in SEQ ID NO: 34. In one specific embodiment, the antibody or the antigen-binding fragment thereof binds to c-Met and comprises at least one (one or more) single variable domain, wherein the single variable domain comprises CDR1, CDR2, and CDR3 of the variable region sequence set forth in SEQ ID NO: 34. In some embodiments, the single variable domain is derived from animals of the family Camelidae. In some embodiments, the single variable domain is humanized. In some embodiments, the single variable domain comprises an acceptor human framework.

In some embodiments, the antibody or the antigen-binding fragment thereof binds to c-Met and comprises at least one (one or more) single variable domain, wherein the single variable domain comprises at least one, at least two, or all three CDRs selected from the following: (a) CDR1 comprising the amino acid sequence set forth in SEQ ID NO: 5, 8, 11, 14, 17, or 20; (b) CDR2 comprising the amino acid sequence set forth in SEQ ID NO: 6, 9, 12, 15, 18, or 21; and (c) CDR3 comprising the amino acid sequence set forth in SEQ ID NO: 7, 10, 13, 16, 19, or 22. In some embodiments, the antibody or the antigen-binding fragment thereof binds to c-Met and comprises at least one (one or more) single variable domain, wherein the single variable domain comprises the following complementarity determining regions: (a) CDR1 comprising the amino acid sequence set forth in SEQ ID NO: 5, 8, 11, 14, 17, or 20; (b) CDR2 comprising the amino acid sequence set forth in SEQ ID NO: 6, 9, 12, 15, 18, or 21; and (c) CDR3 comprising the amino acid sequence set forth in SEQ ID NO: 7, 10, 13, 16, 19, or 22. In some embodiments, the single variable domain is derived from animals of the family Camelidae. In some embodiments, the single variable domain is humanized. In some embodiments, the single variable domain comprises an acceptor human framework.

In some embodiments, the single variable domain comprises: (a) CDR1 comprising the amino acid sequence set forth in SEQ ID NO: 5, CDR2 comprising the amino acid sequence set forth in SEQ ID NO: 6, and CDR3 comprising the amino acid sequence set forth in SEQ ID NO: 7; (b) CDR1 comprising the amino acid sequence set forth in SEQ ID NO: 8, CDR2 comprising the amino acid sequence set forth in SEQ ID NO: 9, and CDR3 comprising the amino acid sequence set forth in SEQ ID NO: 10; (c) CDR1 comprising the amino acid sequence set forth in SEQ ID NO: 11, CDR2 comprising the amino acid sequence set forth in SEQ ID NO: 12, and CDR3 comprising the amino acid sequence set forth in SEQ ID NO: 13; (d) CDR1 comprising the amino acid sequence set forth in SEQ ID NO: 14, CDR2 comprising the amino acid sequence set forth in SEQ ID NO: 15, and CDR3 comprising the amino acid sequence set forth in SEQ ID NO: 16; (e) CDR1 comprising the amino acid sequence set forth in SEQ ID NO: 17, CDR2 comprising the amino acid sequence set forth in SEQ ID NO: 18, and CDR3 comprising the amino acid sequence set forth in SEQ ID NO: 19; or (f) CDR1 comprising the amino acid sequence set forth in SEQ ID NO: 20, CDR2 comprising the amino acid sequence set forth in SEQ ID NO: 21, and CDR3 comprising the amino acid sequence set forth in SEQ ID NO: 22. In some embodiments, the single variable domain is derived from animals of the family Camelidae. In some embodiments, the single variable domain is humanized. In some embodiments, the single variable domain comprises an acceptor human framework.

In some embodiments, the single variable domain comprises: (a) CDR1 of the amino acid sequence set forth in SEQ ID NO: 5, CDR2 of the amino acid sequence set forth in SEQ ID NO: 6, and CDR3 of the amino acid sequence set forth in SEQ ID NO: 7; (b) CDR1 of the amino acid sequence set forth in SEQ ID NO: 8, CDR2 of the amino acid sequence set forth in SEQ ID NO: 9, and CDR3 of the amino acid sequence set forth in SEQ ID NO: 10; (c) CDR1 of the amino acid sequence set forth in SEQ ID NO: 11, CDR2 of the amino acid sequence set forth in SEQ ID NO: 12, and CDR3 of the amino acid sequence set forth in SEQ ID NO: 13; (d) CDR1 of the amino acid sequence set forth in SEQ ID NO: 14, CDR2 of the amino acid sequence set forth in SEQ ID NO: 15, and CDR3 of the amino acid sequence set forth in SEQ ID NO: 16; (e) CDR1 of the amino acid sequence set forth in SEQ ID NO: 17, CDR2 of the amino acid sequence set forth in SEQ ID NO: 18, and CDR3 of the amino acid sequence set forth in SEQ ID NO: 19; or (f) CDR1 of the amino acid sequence set forth in SEQ ID NO: 20, CDR2 of the amino acid sequence set forth in SEQ ID NO: 21, and CDR3 of the amino acid sequence set forth in SEQ ID NO: 22. In some embodiments, the single variable domain is derived from animals of the family Camelidae. In some embodiments, the single variable domain is humanized. In some embodiments, the single variable domain comprises an acceptor human framework. In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24, 26, 28, 30, 32, or 34. In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24, 26, 28, 30, 32, or 34, and the single variable domain comprises the particular CDR1, CDR2, and/or CDR3 sequences described above.

In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24, and the single variable domain comprises: CDR1 comprising the amino acid sequence set forth in SEQ ID NO: 5, CDR2 comprising the amino acid sequence set forth in SEQ ID NO: 6, and CDR3 comprising the amino acid sequence set forth in SEQ ID NO: 7.

In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 26, and the single variable domain comprises: CDR1 comprising the amino acid sequence set forth in SEQ ID NO: 8, CDR2 comprising the amino acid sequence set forth in SEQ ID NO: 9, and CDR3 comprising the amino acid sequence set forth in SEQ ID NO: 10.

In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 28, and the single variable domain comprises: CDR1 comprising the amino acid sequence set forth in SEQ ID NO: 11, CDR2 comprising the amino acid sequence set forth in SEQ ID NO: 12, and CDR3 comprising the amino acid sequence set forth in SEQ ID NO: 13.

In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 30, and the single variable domain comprises: CDR1 comprising the amino acid sequence set forth in SEQ ID NO: 14, CDR2 comprising the amino acid sequence set forth in SEQ ID NO: 15, and CDR3 comprising the amino acid sequence set forth in SEQ ID NO: 16.

In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32, and the single variable domain comprises: CDR1 comprising the amino acid sequence set forth in SEQ ID NO: 17, CDR2 comprising the amino acid sequence set forth in SEQ ID NO: 18, and CDR3 comprising the amino acid sequence set forth in SEQ ID NO: 19.

In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 34, and the single variable domain comprises: CDR1 comprising the amino acid sequence set forth in SEQ ID NO: 20, CDR2 comprising the amino acid sequence set forth in SEQ ID NO: 21, and CDR3 comprising the amino acid sequence set forth in SEQ ID NO: 22.

In some embodiments, a single variable domain amino acid sequence having at least 85%, 86%, 87%, 88%, 89%, 90%, 91%, 92%, 93%, 94%, 95%, 96%, 97%, 98%, 99%, or 100% identity comprises substitutions (e.g., conservative substitutions), insertions, or deletions compared to a reference sequence; however, an antibody or an antigen-binding fragment thereof comprising a single variable domain comprising the sequence retains the ability to bind to c-Met. In some embodiments, a total of 1-18, 1-16, 1-14, 1-12, 1-10, 1-9, 1-8, 1-7, 1-6, 1-5, 1-4, 1-3, or 1-2 amino acids are substituted, inserted and/or deleted in the amino acid sequence selected from SEQ ID NOs: 24, 26, 28, 30, 32, and 34. In some embodiments, the substitutions, insertions or deletions occur in regions outside of CDRs (i.e., in FRs). In some embodiments, the substitutions, insertions or deletions occur in CDR regions, for example, in one, two, or three of CDR1, CDR2, and CDR3. In some embodiments, the substitutions, insertions or deletions occur in CDR regions and non-CDR regions. Optionally, the single variable domain comprises the amino acid sequence selected from SEQ ID NOs: 24, 26, 28, 30, 32, and 34, including post-translational modifications of the sequence.

In some embodiments, the single variable domain comprises the amino acid sequence set forth in SEQ ID NO: 24, 26, 28, 30, 32, or 34.

When the antibody or the antigen-binding fragment thereof comprises two or more single variable domains, identical or different single variable domains may be selected. The antibody may be a monoclonal antibody, a polyclonal antibody, a multispecific antibody (e.g., a bispecific antibody or trispecific antibody), or antibodies of other configurations known in the art. The antigen-binding fragment may be a single variable domain. In one specific embodiment, the antibody is a monospecific antibody. In one specific embodiment, the antibody is a bispecific antibody.

In some embodiments, the single variable domain (including any of the embodiments described above, e.g., the single variable domain described above comprising particular CDR1, CDR2 and/or CDR3) is a $V_HH$. In some specific embodiments, $V_HH$ has the following structure from N-terminus to C-terminus: FR1-CDR1-FR2-CDR2-FR3-CDR3-FR4.

In some embodiments, the single variable domain (including any of the embodiments described above, e.g., the single variable domain described above comprising particular CDR1, CDR2 and/or CDR3) is a humanized $V_HH$. A $V_HH$ of non-human origin may be "humanized" by replacing one or more amino acid residues in the amino acid sequence of the original $V_HH$ with one or more amino acid residues present at corresponding positions in the $V_H$ domain of a human conventional four-chain antibody. Humanization may be desirable to reduce immunogenicity.

In some embodiments, the antibody or the antigen-binding fragment thereof is derived from animals of the family Camelidae. In some embodiments, the antibody or the antigen-binding fragment thereof is chimeric. In some embodiments, the antibody or the antigen-binding fragment thereof comprises an IgG constant region, preferably a human IgG constant region, more preferably a human IgG1 constant region, a human IgG2 constant region, a human IgG3 constant region, or a human IgG4 constant region. In some embodiments, the antibody or the antigen-binding fragment thereof comprises an Fc of IgG. In some embodiments, the antibody or the antigen-binding fragment thereof comprises an Fc of human IgG; preferably, the Fc is an Fc of human IgG1, IgG2, IgG3, or IgG4. In some embodiments, the antibody or the antigen-binding fragment thereof is humanized. In some embodiments, the antibody or the antigen-binding fragment thereof comprises an acceptor human framework.

In some embodiments, the antibody or the antigen-binding fragment thereof comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 37, 39, 41, 43, 45, or 47. In some embodiments, the antibody or the antigen-binding fragment thereof comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 37, 39, 41, 43, 45, or 47, and the antibody or the antigen-binding fragment thereof comprises the particular CDR1, CDR2, and/or CDR3 sequences in the single variable domain described in the present disclosure. In some embodiments, the antibody or the antigen-binding fragment thereof comprises the amino acid sequence set forth in SEQ ID NO: 37, 39, 41, 43, 45, or 47. In some embodiments, the antibody or the antigen-binding fragment thereof comprises at least one polypeptide chain, wherein the amino acid sequence of the at least one polypeptide chain is set forth in SEQ ID NO: 37, 39, 41,43,45, or 47. In some embodiments, the antibody or the antigen-binding fragment thereof comprises two identical polypeptide chains, wherein the amino acid sequence of each of the polypeptide chains is set forth in SEQ ID NO: 37, 39, 41, 43, 45, or 47.

The present disclosure also provides antibodies or antigen-binding fragments thereof that bind to the same epitope as any one of the antibodies or the antigen-binding fragments thereof of the present disclosure. In some specific embodiments, the antibody or the antigen-binding fragment thereof binds to the same epitope as an antibody or an antigen-binding fragment thereof comprising the amino acid sequence set forth in SEQ ID NO: 37, 39, 41,43,45, or 47. In some embodiments, the antibody or the antigen-binding fragment thereof that binds to the same epitope is derived from animals of the family Camelidae, is chimeric or is humanized. Based on the binding to the same epitope, competitive screening of antibodies or antigen-binding fragments thereof can be carried out using conventional techniques known to those skilled in the art. For example, competitive studies can be carried out to obtain antibodies or antigen-binding fragments thereof that compete with each other for binding to antigens. Thus, the present disclosure also provides an antibody or an antigen-binding fragment thereof that competes for binding to c-Met with any one of the antibodies or the antigen-binding fragments thereof described in the present disclosure. In some specific embodiments, the antibody or the antigen-binding fragment thereof competes for binding to c-Met with an antibody or an antigen-binding fragment thereof comprising the amino acid sequence set forth in SEQ ID NO: 37, 39, 41, 43, 45, or 47. The binding to c-Met can be measured by ELISA, flow cytometry or surface plasmon resonance (SPR) assay, or any other method known in the art. In some embodiments, the antibody or the antigen-binding fragment thereof that competes for binding to c-Met is derived from animals of the family Camelidae, is chimeric or is humanized.

In some embodiments, the antibody or the antigen-binding fragment thereof according to any one of the embodiments described above may comprise one or several of the following properties and characteristics, alone or in combination:

(i) binding to human c-Met or/and monkey c-Met;
(ii) blocking the binding of HGF or a β chain thereof to c-Met;
(iii) inhibiting c-Met phosphorylation caused by HGF stimulation;
(iv) exerting the antibody-dependent cell-mediated cytotoxicity (ADCC) effect on tumor cells.

In some embodiments, the antibody or the antigen-binding fragment thereof binds to c-Met, e.g., monkey c-Met and/or human c-Met. In some embodiments, the antibody or the antigen-binding fragment thereof binds to cynomolgus monkey c-Met. In some embodiments, the antibody or the antigen-binding fragment thereof binds to human c-Met. In some embodiments, the antibody or the antigen-binding fragment thereof has the following binding affinity ($K_D$) for human c-Met: in the range of about 1E-11 M to about 1E-07 M, about 1E-10 M to about 1E-08 M, or about 2.00E-10 M to about 8.25E-09 M. In some embodiments, the antibody or the antigen-binding fragment thereof has the following binding affinity ($K_D$) for human c-Met: about 1E-07 M or less, about 1E-08 M or less, about 8.25E-09 M or less, about 1E-09 M or less, about 2.00E-10 M or less, about 1E-10 M or less, or about 1E-11 M or less. In some embodiments, the binding affinity $K_D$ of the antibody or the antigen-binding fragment thereof provided by the present disclosure is measured by Biacore. In some embodiments, the antibody or the antigen-binding fragment thereof blocks the binding of HGF or a β chain thereof to c-Met.

In some embodiments, the antibody or the antigen-binding fragment thereof inhibits c-Met phosphorylation caused by HGF stimulation.

In some embodiments, the antibody or the antigen-binding fragment thereof exerts the ADCC effect on tumor cells. In some embodiments, the tumor cell is a c-Met expressing tumor cell.

The present disclosure provides exemplary antibodies or antigen-binding fragments thereof, such as monospecific antibodies (including chimeric antibodies 1B-3B11-Fc, 1B-1B6-Fc, 1B-1C7-Fc, 1B-1A8-Fc, 1B-1B2-Fc, and 3B-1C7-Fc). The single variable domain is fused with human IgG1, forming a homodimer through Fc. The amino acid sequences of the exemplary antibodies are provided in Table S3 below.

Table S3. Full-length sequences of exemplary antibodies

| Name | Amino acid sequence | SEQ ID NO: |
|---|---|---|
| 1B-3B11-Fc | QVQLVESGGGSVQAGGSLRLSCVVSGYTYSSVAWFRQTP GKEREGVAVINPGGDNTDYTYAVKGRFTISRDSAKSTVFL QMNNLESEDTAMYYCAAGRTFGGTWMDRNKWKYWGQ GTLVTVSSEPKIPQPQPKPQPQPQPQPKPQPKPEPECTCPK CPAPELLGGPSVFLFPPKPKDTLMISRTPEVTCVVVDVSHE DPEVKFNWYVDGVEVHNAKTKPREEQYNSTYRVVSVLT VLHQDWLNGKEYKCKVSNKALPAPIEKTISKAKGQPREP QVYTLPPSRDELTKNQVSLTCLVKGFYPSDIAVEWESNGQ PENNYKTTPPVLDSDGSFFLYSKLTVDKSRWQQGNVFSC SVMHEALHNHYTQKSLSLSPGK | 37 |
| 1B-1B6-Fc | QVQLVESGGDSVQAGGSLRLSCAASAHAYSSGCMGWFR QAPGKGREGVATIYTRNDFTYYSDSVKGRFTISRDDAKN TVYLQVNSLKAEDTAMYYCAAGSPFRYAGAWNVCGLKE EDYNYWGQGTLVTVSSEPKIPQPQPKPQPQPQPQPKPQPK PEPECTCPKCPAPELLGGPSVFLFPPKPKDTLMISRTPEVT CVVVDVSHEDPEVKFNWYVDGVEVHNAKTKPREEQYN STYRVVSVLTVLHQDWLNGKEYKCKVSNKALPAPIEKTIS KAKGQPREPQVYTLPPSRDELTKNQVSLTCLVKGFYPSDI AVEWESNGQPENNYKTTPPVLDSDGSFFLYSKLTVDKSR WQQGNVFSCSVMHEALHNHYTQKSLSLSPGK | 39 |
| 1B-1C7-Fc | QVQLVESGGGLVQPGGSLRLSCAASGYSYSANCVGWFY QAPGKEREGVAAINTGGETATYADFVKGRFTISQDYAKNT VYLQMNSLKSEDTGMYYCAAVGTRKYWDECSLSQHAY KNWGQGTLVTVSSEPKIPQPQPKPQPQPQPQPKPQPKPEP ECTCPKCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSRDELTKNQVSLTCLVKGFYPSDIAVE WESNGQPENNYKTTPPVLDSDGSFFLYSKLTVDKSRWQQ GNVFSCSVMHEALHNHYTQKSLSLSPGK | 41 |
| 1B-1A8-Fc | EVQLVESGGGLVQPGGSLRLSCVASGFTFKSYAMDWVRQ APGKGLEWIAGINSAGSPSYEASVKGRFTISRDNAENTLY LQLNSLKTEDTAMYYCTKCRDFRSPYCRPGQGTLVTVSS EPKIPQPQPKPQPQPQPQPKPQPKPEPECTCPKCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SRDELTKNQVSLTCLVKGFYPSDIAVEWESNGQPENNYKT TPPVLDSDGSFFLYSKLTVDKSRWQQGNVFSCSVMHEAL HNHYTQKSLSLSPGK | 43 |
| 1B-1B2-Fc | EVQLVESGGGSVQAGGSLRLSCTASGFTFDMGWYRQAP GDECELVSSISSDGNSDYLDSVKGRFTISQDNAKNTVYLQ MNSLKPEDTAVYYCAANGAGHLVLRPLCGSNAVDAWGQ GTLVTVSSEPKIPQPQPKPQPQPQPQPKPQPKPEPECTCPK CPAPELLGGPSVFLFPPKPKDTLMISRTPEVTCVVVDVSHE DPEVKFNWYVDGVEVHNAKTKPREEQYNSTYRVVSVLT VLHQDWLNGKEYKCKVSNKALPAPIEKTISKAKGQPREP QVYTLPPSRDELTKNQVSLTCLVKGFYPSDIAVEWESNGQ PENNYKTTPPVLDSDGSFFLYSKLTVDKSRWQQGNVFSC SVMHEALHNHYTQKSLSLSPGK | 45 |

(continued)

| Name | Amino acid sequence | SEQ ID NO: |
|------|---------------------|------------|
| 3B-1C7-Fc | QVQLVESGGDSVQAGGSLRLSCIASGFTFDGTTMGWYRQ APGDECELVSDISSDGNTYYSDSVKGRFTISQGNAKNTVY LQMNSLTPEDTAVYYCAAAEFRLLLRPLCRQDDYGYWG QGTLVTVSSEPKIPQPQPKPQPQPQPQPKPQPKPEPECTCP KCPAPELLGGPSVFLFPPKPKDTLMISRTPEVTCVVVDVS HEDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGK | 47 |

*Fusion protein*

The single variable domains, and the antibodies or the antigen-binding fragments thereof of the present disclosure can be used to construct any desired fusion protein, thereby imparting to the fusion protein the property of targeted binding to c-Met or other properties that single variable domains, antibodies or antigen-binding fragments thereof have. Accordingly, the present disclosure provides an isolated fusion protein comprising at least one (one or more) of the single variable domains, or the antibodies or the antigen-binding fragments thereof of the present disclosure.

In some specific embodiments, the fusion protein comprises the exemplary CDR sequence, single variable domain sequence, or antibody sequence of the present disclosure, e.g., one, two, or three CDR sequences in any single variable domain in Table S1, any single variable domain sequence in Table S2, or any antibody sequence in Table S3; optionally, the fusion protein may be subjected to any modification known in the art (e.g., glycosylation modification or chemical modification).

When the fusion protein comprises two or more single variable domains, identical or different single variable domains may be selected.

*Pharmaceutical composition*

The present disclosure provides a pharmaceutical composition comprising the antibody or the antigen-binding fragment thereof of the present disclosure, and a pharmaceutically acceptable carrier. The present disclosure also provides a pharmaceutical composition comprising the fusion protein of the present disclosure, and a pharmaceutically acceptable carrier. In some embodiments, any one or more of the 1B-3B11-Fc, 1B-1B6-Fc, 1B-1C7-Fc, 1B-1A8-Fc, 1B-1B2-Fc, and 3B-1C7-Fc antibodies, and a pharmaceutically acceptable carrier are formed into a pharmaceutical composition.

*Isolated nucleic acid*

The present disclosure provides an isolated nucleic acid encoding the single variable domain, or the antibody or the antigen-binding fragment thereof described in the present disclosure. The present disclosure also provides an isolated nucleic acid encoding the fusion protein described in the present disclosure. In some embodiments, the nucleic acid encodes a single variable domain, such as the single variable domain of 1B-3B11-Fc, 1B-1B6-Fc, 1B-1C7-Fc, 1B-1A8-Fc, 1B-1B2-Fc, or 3B-1C7-Fc. In some embodiments, the nucleic acid encodes an antibody or an antigen-binding fragment thereof, such as 1B-3B11-Fc, 1B-1B6-Fc, 1B-1C7-Fc, 1B-1A8-Fc, 1B-1B2-Fc, or 3B-1C7-Fc.

By way of example, the nucleic acid sequences of some single variable domains, antibodies or antigen-binding fragments thereof are listed in the sequence listing.

*Vector*

The present disclosure provides a vector comprising the isolated nucleic acid. In some embodiments, the vector is a cloning vector; in some other embodiments, the vector is an expression vector. The expression vector is optionally any expression vector capable of expressing the single variable domain, or the antibody or the antigen-binding fragment thereof described in the present disclosure. The expression vector is optionally any expression vector capable of expressing the fusion protein described in the present disclosure.

As a specific example, the expression vector is pcDNA3.1.

*Host cell*

The present disclosure provides a host cell comprising the nucleic acid or the vector described in the present disclosure, the host cell being a suitable host cell for cloning or expressing a single variable domain, or an antibody or an antigen-binding fragment thereof. The present disclosure also provides a host cell comprising the nucleic acid or the vector described in the present disclosure, the host cell being a suitable host cell for cloning or expressing a fusion protein. In some embodiments, the host cell is a prokaryotic cell. In other embodiments, the host cell is a eukaryotic cell. In some embodiments, the host cell is selected from a yeast cell, a mammalian cell, and other cells suitable for preparing a single variable domain, an antibody or an antigen-binding fragment thereof, or a fusion protein. Mammalian cells are, for example, Chinese hamster ovary (CHO) cells or CHO-S cells.

*Methods for preparing single variable domain, antibody or αntigen-binding fragment thereof, or fusion protein*

The present disclosure provides a method for preparing a single variable domain, which comprises: culturing a host cell comprising a nucleic acid encoding the single variable domain of the present disclosure, and recovering the single variable domain from the host cell or host cell culture medium. The present disclosure also provides a method for preparing an antibody or an antigen-binding fragment thereof, which comprises: culturing a host cell comprising a nucleic acid encoding the antibody or the antigen-binding fragment thereof of the present disclosure, and recovering the antibody or the antigen-binding fragment thereof from the host cell or host cell culture medium. The present disclosure also provides a method for preparing an fusion protein, which comprises: culturing a host cell comprising a nucleic acid encoding the fusion protein of the present disclosure, and recovering the fusion protein from the host cell or host cell culture medium.

To produce the single variable domain, or the antibody or the antigen-binding fragment thereof, a nucleic acid encoding the single variable domain, the antibody or the antigen-binding fragment thereof is inserted into a vector for further cloning or/and expression in a host cell. To produce the fusion protein, a nucleic acid encoding the fusion protein is inserted into a vector for further cloning or/and expression in a host cell. The nucleic acid can be obtained using various methods known in the art, such as gene splicing and chemical synthesis.

*Use*

The present disclosure provides use of the single variable domain, the antibody or the antigen-binding fragment thereof, or the fusion protein of the present disclosure. The present disclosure also provides use of the pharmaceutical composition of the present disclosure.

The present disclosure provides use of the single variable domain of the present disclosure for preparing a medicament for treating a subject having a tumor. The present disclosure also provides use of the antibody or the antigen-binding fragment thereof of the present disclosure for preparing a medicament for treating a subject having a tumor. The present disclosure also provides use of the fusion protein of the present disclosure for preparing a medicament for treating a subject having a tumor. The present disclosure also provides use of the pharmaceutical composition of the present disclosure for preparing a medicament for treating a subject having a tumor.

The present disclosure provides a method for detecting or measuring c-Met in a sample, comprising contacting the sample with the single variable domain, or the antibody or the antigen-binding fragment thereof of the present disclosure, and detecting or measuring a binding complex. The present disclosure also provides a method for detecting or measuring c-Met in a sample, comprising contacting the sample with the fusion protein of the present disclosure, and detecting or measuring a binding complex.

The present disclosure provides a method for inhibiting, reducing or blocking c-Met signaling in a cell, which comprises administering to the cell an effective amount of the single variable domain, or the antibody or the antigen-binding fragment thereof described in the present disclosure. The present disclosure also provides use of the single variable domain, or the antibody or the antigen-binding fragment thereof of the present disclosure for preparing a medicament for inhibiting, reducing or blocking c-Met signaling in a cell. The present disclosure provides a method for inhibiting, reducing or blocking c-Met signaling in a cell, comprising administering to the cell an effective amount of the fusion protein described in the present disclosure. The present disclosure also provides use of the fusion protein of the present disclosure for preparing a medicament for inhibiting, reducing or blocking c-Met signaling in a cell. The present disclosure provides a method for inhibiting, reducing or blocking c-Met signaling in a cell, which comprises administering to the cell an effective amount of the pharmaceutical composition described in the present disclosure. The present disclosure also provides use of the pharmaceutical composition of the present disclosure for preparing a medicament for inhibiting, reducing or blocking c-Met signaling in a cell. In some embodiments, the cell is a tumor cell.

The present disclosure provides a method for inhibiting tumor cell growth or killing a tumor cell, comprising administering to the tumor cell an effective amount of the single variable domain, or the antibody or the antigen-binding fragment thereof of the present disclosure. The present disclosure also provides use of the single variable domain, or the antibody or the antigen-binding fragment thereof for preparing a medicament for inhibiting tumor cell growth or killing a tumor cell. The present disclosure provides a method for inhibiting tumor cell growth or killing a tumor cell, comprising administering to the tumor cell an effective amount of the fusion protein of the present disclosure. The present disclosure also provides use of the fusion protein for preparing a medicament for inhibiting tumor cell growth or killing a tumor cell. The present disclosure provides a method for inhibiting tumor cell growth or killing a tumor cell, comprising administering to the tumor cell an effective amount of the pharmaceutical composition of the present disclosure. The present disclosure also provides use of the pharmaceutical composition for preparing a medicament for inhibiting tumor cell growth or killing a tumor cell.

The present disclosure provides a method for treating a subject having a tumor, comprising administering to the subject a therapeutically effective amount of the single variable domain, or the antibody or the antigen-binding fragment thereof described in the present disclosure. The present disclosure also provides a method for treating a subject having a tumor, comprising administering to the subject a therapeutically effective amount of the fusion protein described in the present disclosure. The present disclosure also provides a method for treating a subject having a tumor, comprising administering to the subject a therapeutically effective amount of the pharmaceutical composition described in the present disclosure. Subjects in need of treatment include those with a disease or condition, as well as those who may have the disease or condition and whose purpose is to prevent, delay or alleviate the disease or condition.

In some embodiments, the tumor described above is a c-Met expressing tumor. In some embodiments, the tumor described above is a c-Met overexpressing tumor. In some embodiments, the tumor described above is a solid tumor. In some embodiments, the tumor described above is a non-solid tumor. In some embodiments, the tumor described above is brain cancer, head and neck cancer, lung cancer, esophageal cancer, pharyngeal cancer, nasal cancer, tongue cancer, oral cancer, thyroid cancer, skin cancer, bone cancer, soft tissue sarcoma, myeloma, gastric cancer, gastroesophageal junction adenocarcinoma, breast cancer, liver cancer, kidney cancer, pancreatic cancer, spleen cancer, lymphoma, thymus cancer, bladder cancer, vaginal cancer, testicular cancer, uterine cancer, cervical cancer, colorectal cancer, anal cancer, papillary carcinoma, prostate cancer, ovarian cancer, epithelial cell cancer, or hematologic tumor. In some embodiments, the lung cancer is non-small cell lung cancer. In some embodiments, the brain cancer is glioblastoma.

The present disclosure further provides the following specific embodiments, which, however, are not intended to limit the scope of the present invention:

Embodiment 1. An isolated antibody or an antigen-binding fragment thereof, wherein the antibody or the antigen-binding fragment thereof binds to c-Met and comprises at least one single variable domain, wherein the single variable domain comprises the following complementarity determining regions:

a) CDR1 comprising the amino acid sequence set forth in SEQ ID NO: 5, 8, 11, 14, 17, or 20;
b) CDR2 comprising the amino acid sequence set forth in SEQ ID NO: 6, 9, 12, 15, 18, or 21; and
c) CDR3 comprising the amino acid sequence set forth in SEQ ID NO: 7, 10, 13, 16, 19, or 22.

Embodiment 2. The isolated antibody or the antigen-binding fragment thereof according to Embodiment 1, wherein the single variable domain comprises:

a) CDR1 comprising the amino acid sequence set forth in SEQ ID NO: 5, CDR2 comprising the amino acid sequence set forth in SEQ ID NO: 6, and CDR3 comprising the amino acid sequence set forth in SEQ ID NO: 7;
b) CDR1 comprising the amino acid sequence set forth in SEQ ID NO: 8, CDR2 comprising the amino acid sequence set forth in SEQ ID NO: 9, and CDR3 comprising the amino acid sequence set forth in SEQ ID NO: 10;
c) CDR1 comprising the amino acid sequence set forth in SEQ ID NO: 11, CDR2 comprising the amino acid sequence set forth in SEQ ID NO: 12, and CDR3 comprising the amino acid sequence set forth in SEQ ID NO: 13;
d) CDR1 comprising the amino acid sequence set forth in SEQ ID NO: 14, CDR2 comprising the amino acid sequence set forth in SEQ ID NO: 15, and CDR3 comprising the amino acid sequence set forth in SEQ ID NO: 16;
e) CDR1 comprising the amino acid sequence set forth in SEQ ID NO: 17, CDR2 comprising the amino acid sequence set forth in SEQ ID NO: 18, and CDR3 comprising the amino acid sequence set forth in SEQ ID NO: 19; or
f) CDR1 comprising the amino acid sequence set forth in SEQ ID NO: 20, CDR2 comprising the amino acid sequence set forth in SEQ ID NO: 21, and CDR3 comprising the amino acid sequence set forth in SEQ ID NO: 22.

Embodiment 3. The isolated antibody or the antigen-binding fragment thereof according to Embodiment 2, wherein the single variable domain comprises an amino acid sequence having at least 85% identity to the amino acid sequence set forth in SEQ ID NO: 24, 26, 28, 30, 32, or 34.

Embodiment 4. The isolated antibody or the antigen-binding fragment thereof according to Embodiment 3, wherein the single variable domain comprises the amino acid sequence set forth in SEQ ID NO: 24, 26, 28, 30, 32, or 34.

Embodiment 5. The isolated antibody or the antigen-binding fragment thereof according to any one of Embodiments 1 to 4, wherein the single variable domain is a $V_HH$.

Embodiment 6. The isolated antibody or the antigen-binding fragment thereof according to any one of Embodiments 1 to 5, wherein the antibody or the antigen-binding fragment thereof is chimeric or humanized.

Embodiment 7. The isolated antibody or the antigen-binding fragment thereof according to any one of Embodiments 1 to 6, wherein the antibody or the antigen-binding fragment thereof comprises an Fc of human IgG, preferably an Fc of human IgG1, IgG2, IgG3, or IgG4.

Embodiment 8. The isolated antibody or the antigen-binding fragment thereof according to any one of Embodiments 1 to 7, wherein the antibody or the antigen-binding fragment thereof comprises the amino acid sequence set forth in SEQ ID NO: 37, 39, 41, 43, 45, or 47.

Embodiment 9. The isolated antibody or the antigen-binding fragment thereof according to any one of Embodiments 1 to 8, wherein the antibody or the antigen-binding fragment thereof may comprise one or several of the following properties and characteristics, alone or in combination:

Embodiment 10. binding to human c-Met or/and monkey c-Met;
Embodiment 11. blocking the binding of HGF or a β chain thereof to c-Met;
Embodiment 12. inhibiting c-Met phosphorylation caused by HGF stimulation;
Embodiment 13. exerting the ADCC effect on tumor cells.

Embodiment 14. A fusion protein comprising the antibody or the antigen-binding fragment thereof according to any one of Embodiments 1 to 9.

Embodiment 15. A pharmaceutical composition comprising the antibody or the antigen-binding fragment thereof according to any one of Embodiments 1 to 9, or the fusion protein according to Embodiment 10, and a pharmaceutically acceptable carrier.

Embodiment 16. An isolated nucleic acid encoding the antibody or the antigen-binding fragment thereof according to any one of Embodiments 1 to 9 or the fusion protein according to Embodiment 10.

Embodiment 17. A vector comprising the isolated nucleic acid according to Embodiment 12.

Embodiment 18. A host cell comprising the nucleic acid according to Embodiment 12 or the vector according to Embodiment 13.

Embodiment 19. A method for preparing the isolated antibody or the antigen-binding fragment thereof according to any one of Embodiments 1 to 9, comprising: culturing the host cell according to Embodiment 14 and isolating the antibody or the antigen-binding fragment thereof expressed, wherein the vector is an expression vector.

Embodiment 20. Use of the isolated antibody or the antigen-binding fragment thereof according to any one of Embodiments 1 to 9 for preparing a medicament for treating a subject having a tumor.

Embodiment 21. Use of the fusion protein according to Embodiment 10 for preparing a medicament for treating a subject having a tumor.

Embodiment 22. Use of the pharmaceutical composition according to Embodiment 11 for preparing a medicament for treating a subject having a tumor.

Embodiment 23. The use according to any one of Embodiments 16 to 18, wherein the tumor is brain cancer, head and neck cancer, lung cancer, esophageal cancer, pharyngeal cancer, nasal cancer, tongue cancer, oral cancer, thyroid cancer, skin cancer, bone cancer, soft tissue sarcoma, myeloma, gastric cancer, gastroesophageal junction adenocarcinoma, breast cancer, liver cancer, kidney cancer, pancreatic cancer, spleen cancer, lymphoma, thymus cancer, bladder cancer, vaginal cancer, testicular cancer, uterine cancer, cervical cancer, colorectal cancer, anal cancer, papillary carcinoma, prostate cancer, ovarian cancer, epithelial cell cancer, or hematologic tumor.

Embodiment 24. A method for detecting or measuring c-Met in a sample, comprising contacting the sample with the antibody or the antigen-binding fragment thereof according to any one of Embodiments 1 to 9, and detecting or measuring a binding complex.

Embodiment 25. A method for inhibiting, reducing or blocking c-Met signaling in a cell, comprising administering to the cell an effective amount of the antibody or the antigen-binding fragment thereof according to any one of Embodiments 1 to 9, the fusion protein according to Embodiment 10, or the pharmaceutical composition according to Embodiment 11.

Embodiment 26. A method for inhibiting tumor cell growth or killing a tumor cell, comprising administering to the tumor cell an effective amount of the antibody or the antigen-binding fragment thereof according to any one of Embodiments 1 to 9, the fusion protein according to Embodiment 10, or the pharmaceutical composition according to Embodiment 11.

Embodiment 27. A method for treating a subject having a tumor, comprising administering to the subject a therapeutically effective amount of the antibody or the antigen-binding fragment thereof according to any one of Embodiments 1 to 9, the fusion protein according to Embodiment 10, or the pharmaceutical composition according to Embodiment 11.

Embodiment 28. The method according to Embodiment 22 or 23, wherein the tumor is brain cancer, head and neck cancer, lung cancer, esophageal cancer, pharyngeal cancer, nasal cancer, tongue cancer, oral cancer, thyroid cancer, skin cancer, bone cancer, soft tissue sarcoma, myeloma, gastric cancer, gastroesophageal junction adenocarcinoma, breast cancer, liver cancer, kidney cancer, pancreatic cancer, spleen cancer, lymphoma, thymus cancer, bladder cancer, vaginal cancer, testicular cancer, uterine cancer, cervical cancer, colorectal cancer, anal cancer, papillary carcinoma, prostate cancer, ovarian cancer, epithelial cell cancer, or hematologic tumor.

Although the aforementioned invention has been described in considerable detail by providing illustrations and examples for the purpose of clear understanding, it will be apparent to those of ordinary skills in the art in light of the teachings of the present disclosure that certain changes and modifications can be made to the present disclosure without departing from the spirit or scope of the appended claims. The following examples are provided for illustrative purposes only and are not intended to be limiting. Those skilled in the art will readily identify a variety of noncritical parameters that may be changed or modified to produce substantially similar results.

Example 1. Construction of Anti-c-Met $V_H$H Phage Display Library

1.1 Animal immunization

A recombinant human c-Met-Fc fusion protein (SinoBiological, product catalog No. 10692-H02H) was mixed and emulsified with complete Freund's adjuvant in a ratio by volume of 1:1. A bactrian camel was first immunized by subcutaneous multipoint injection, and then immunized with the recombinant human c-Met-Fc fusion protein mixed and emulsified with incomplete Freund's adjuvant in a ratio by volume of 1:1 as a booster at intervals of 2 weeks. The serum was collected after the fourth or fifth immunization and assayed for anti-human c-Met antibody titer. After multiple rounds of immunization, the peripheral blood of the bactrian camel was collected, and peripheral blood mononuclear cells (PBMCs) were isolated.

1.2 RNA extraction

The total RNA was extracted from the PBMCs (from 1.1) using TRIzol™ reagent. The quality of the extracted total RNA was assessed by 1% agarose gel electrophoresis. Quantification was performed by measuring absorbance at 260 nm and 280 nm. The ratio $OD_{260\,nm}/OD_{280\,nm}$ should fall somewhere between 1.8 and 2.0.

1.3 $V_H$H amplification

The total RNA was reverse-transcribed into cDNA using the cDNA synthesis kit PrimeScript™ II 1st Strand cDNA Synthesis Kit (TAKARA, product catalog No. 6210A) by following the instructions. The variable region ($V_H$H) sequence of the camel antibody was amplified by nested PCR, specifically as follows: With cDNA as a template, primers Call001 (SEQ ID NO: 1) and Call002 (SEQ ID NO: 2) were used to perform the first round of PCR amplification. The DNA product obtained in short fragments from the amplification was purified using a gel recovery kit (QIAGEN, product catalog No. 28706). With the product of the first round of PCR as a template, primers V-Back (SEQ ID NO: 3) and V-Fwd (SEQ ID NO: 4) were used to perform the second round of PCR amplification. The DNA product obtained from the amplification was the $V_H$H coding fragment, which was then purified using a gel recovery kit (QIAGEN, product catalog No. 28706).

Call001 (SEQ ID NO: 1): GTCCTGGCTGCTCTTCTACAAGG
Call002 (SEQ ID NO: 2): GGTACGTGCTGTTGAACTGTTCC
V-Back (SEQ ID NO: 3): GATGTGCAGCTGCAGGAGTCTGGRGGAGG
V-Fwd (SEQ ID NO: 4): CTAGTGCGGCCGCTGAGGAGACGGTGACCTGGGT

1.4 Construction of $V_H$H phage library

The $V_H$H coding fragment obtained from the amplification was digested with PstIINotI endonuclease and inserted into a phagemid vector pMECS (NTCC Plasmid Vector, Strain, Cell and Gene Collection, product catalog No. pMECS) to construct a recombinant vector, which was then electrically transferred into *Escherichia coli* TG1 (Lucigen, product catalog No. 60502-1). A small portion of the transformed bacterial liquid was diluted and then applied to a selective plate containing 100 μg/mL ampicillin. The library capacity was calculated by colony counting, and 100 random clones were picked for sequencing to assess the library quality. The remaining bacterial liquid was applied to a selective plate containing 100 μg/mL ampicillin. The bacterial lawn of bacterial colonies were scraped off from the plate, supplemented with glycerol and cryopreserved at -80°C as a library stock. The library stock was amplified to logarithmic growth phase. Library amplification was performed by adding M13KO7 helper phages (New England Biolabs, product catalog No. N0315S), with shaking overnight at 200 rpm at 28°C. The bacterial liquid was centrifuged, and the supernatant was collected. 1/4 supernatant volume of a PEG6000/NaCl solution (20% PEG6000 (w/v), 2.5 M NaCl) was added. The mixture was incubated on ice for 1-2 h to settle the phages. The settled phages were centrifuged and collected, resuspended in PBS, and then 20% glycerol was added. The suspension was preserved at -80°C as a $V_HH$ phage display library.

Example 2. Anti-Human c-Met $V_HH$ Screening

2.1 Panning

The $V_HH$ phage display library was subjected to solid-phase panning. The recombinant c-Met-Avitage™ (ACRO, product catalog No. MET-H82E1) was fixed on a high-adsorption microplate. After blocking, the phages obtained in 1.4 were added to the well plate, and the plate was incubated at 37°C for 1-2 h. The plate was washed 10 times with phosphate-buffered saline with Tween (PBST) to remove non-specifically bound phage. After washing with PBS, the bound phages were eluted with trypsin. The enzyme was neutralized with 4-(2-aminoethyl)benzenesulfonyl fluoride hydrochloride (AEBSF), and the eluted phages were used to infect *Escherichia coli* TG1. After amplification, another round of library panning was carried out. After 2-3 rounds of panning and enrichment, the collected phages were used to infect *Escherichia coli* TG1 in logarithmic growth phase. The bacteria was applied to a selective plate containing 20% (w/v) glucose and 100 μg/mL ampicillin. Single clones were picked and cultured. Expression was induced by isopropyl-β-D-thiogalactoside (IPTG), and the supernatant was collected.

2.2 Positive clone identification

The picked clones were subjected to positive identification by indirect ELISA for human c-Met-His (SinoBiological, product catalog No. 10692-H08H). Human c-Met-His or a control protein human Fc protein (ACRO, product catalog No. FCC-H5214) was allowed to coat a high-adsorption microplate, and then the plate was blocked with a blocking buffer. The supernatant prepared in 2.1 was added, and the plate was incubated at 37°C for 1 h. After the plate was washed, an HRP-conjugated anti-HA tag secondary antibody (GenScript, product catalog No. A01296) was added. The plate was incubated at 37°C for 0.5 h and then washed 5 times, and a chromogenic substrate was added for color development. The light absorption signal values at the wavelength of 450 nm and the reference wavelength of 650 nm were measured. Positive clones that bind only to human c-Met-His with relatively high signal values were selected for preservation and sequenced.

Positive clones 1B-1B2, 1B-3B11, 1B-1C7, 1B-1B6, 1B-1A8, and 3B-1C7 were obtained from the screening. The sequence analysis shows that the $V_HH$ of 1B-3B11 has a nucleotide sequence set forth in SEQ ID NO: 23, and has the amino acid sequence set forth in SEQ ID NO: 24; the $V_HH$ of 1B-1B6 has a nucleotide sequence set forth in SEQ ID NO: 25, and has the amino acid sequence set forth in SEQ ID NO: 26; the $V_HH$ of 1B-1C7 has a nucleotide sequence set forth in SEQ ID NO: 27, and has the amino acid sequence set forth in SEQ ID NO: 28; the $V_HH$ of 1B-1A8 has a nucleotide sequence set forth in SEQ ID NO: 29, and has the amino acid sequence set forth in SEQ ID NO: 30; the $V_HH$ of 1B-1B2 has a nucleotide sequence set forth in SEQ ID NO: 31, and has the amino acid sequence set forth in SEQ ID NO: 32; the $V_HH$ of 3B-1C7 has a nucleotide sequence set forth in SEQ ID NO: 33, and has the amino acid sequence set forth in SEQ ID NO: 34.

Example 3. Preparation of Anti-Human c-Met $V_HH$-Fc Chimeric Antibodies

3.1 Preparation of $V_HH$-$F_C$ chimeric antibodies

The $V_HH$ sequences of the positive clones obtained from the screening were linked to a human Fc region to construct $V_HH$-$F_C$ chimeric antibodies. Specifically, the $V_HH$ sequences sequenced in 2.2 were inserted into a pCDNA3.1 eukaryotic expression vector containing a human IgG1 Fc region. These $V_HH$-$F_C$ chimeric antibodies were expressed using an Expifectamine™ CHO Transfection Kit transient expression system (Thermo Fisher Scientific Inc., product catalog No. A29129).

The sequence analysis shows that full-length of 1B-3B11-Fc has the amino acid sequence set forth in SEQ ID NO: 37, and has a nucleotide sequence set forth in SEQ ID NO: 36; full-length of 1B-1B6-Fc has the amino acid sequence set forth in SEQ ID NO: 39, and has a nucleotide sequence set forth in SEQ ID NO: 38; full-length of 1B-1C7-Fc has the amino acid sequence set forth in SEQ ID NO: 41, and has a nucleotide sequence set forth in SEQ ID NO: 40; full-length of 1B-1A8-Fc has the amino acid sequence set forth in SEQ ID NO: 43, and has a nucleotide sequence set forth in SEQ ID NO: 42; full-length of 1B-1B2-Fc has the amino acid sequence set forth in SEQ ID NO: 45, and has a nucleotide sequence set forth in SEQ ID NO: 44; full-length of 3B-1C7-Fc has the amino acid sequence set forth in SEQ ID NO: 47, and has a nucleotide sequence set forth in SEQ ID NO: 46.

3.2 Determination of affinity of $V_H$H-$F_C$ chimeric antibodies for human c-Met and cynomolgus monkey c-Met by surface plasmon resonance

The anti-human c-Met $V_H$H-Fc chimeric antibodies were tested for affinity using a biomolecular interaction analysis system (GE, Biacore T200). An anti-hIgG (Fc) antibody (GE, product catalog No. BR-1008-39) was amino-coupled to a CM5 sensor chip. The anti-human c-Met $V_H$H-Fc chimeric antibodies were diluted to 1 $\mu$g/mL with a running buffer (137 mM NaCl, 2.7 mM KCl, 10 mM $Na_2HPO_4 \cdot 12H_2O$, 1.8 mM $KH_2PO_4$, 0.05% surfactant P-20 (w/v), pH 7.4), and capture was allowed by passing the dilutions through experimental channels at a flow rate of 30 $\mu$L/min. Human c-Met-His (SinoBiological, product catalog No. 10692-H08H) or cynomolgus monkey c-Met-His (SinoBiological, catalog No. 90304-C08H) was diluted with the running buffer to 100 nM, 50 nM, 25 nM, 12.5 nM, 6.25 nM and 3.125 nM, and binding was allowed for 200 s by passing the dilutions at a flow rate of 50 $\mu$L/min. Then dissociation was allowed for 800 s by stopping sample injection. The data signals were collected by BiaControl Software 2.0 in real time, and analyzed by BiaEvaluation Software 2.0. Fitting was performed using a Langmuir 1:1 model, and the binding rate constant $K_a$ (1/Ms), the dissociation rate constant $K_d$ (1/s) and the equilibrium dissociation constant $K_D$ (M) were calculated. The results are shown in Table 1. 1B-1B2-Fc, 1B-3B11-Fc, 1B-1C7-Fc, 1B-1B6-Fc, 1B-1A8-Fc and 3B-1C7-Fc all have relatively high affinity for human c-Met protein, and all have cross-reactions with cynomolgus monkey c-Met protein.

Table 1. Affinity of VH-Fc chimeric antibodies for human c-Met and cynomolgus monkey c-Met

| $V_H$H-Fc Chimeric antibodies | Human c-Met-his | | | Cynomolgus monkey c-Met-His | | |
|---|---|---|---|---|---|---|
| | $K_a$ (1/Ms) | $K_d$ (1/s) | $K_D$ (M) | $K_a$ (1/Ms) | $K_d$ (1/s) | $K_D$ (M) |
| 1B-3B11-Fc | 5.03E+04 | 6.81E-05 | 1.35E-09 | 4.35E+04 | 2.88E-05 | 6.64E-10 |
| 1B-1B2-Fc | 1.41E+05 | 5.60E-04 | 3.99E-09 | 7.84E+04 | 2.15E-04 | 2.74E-09 |
| 1B-1C7-Fc | 2.21E+05 | 4.43E-05 | 2.00E-10 | 2.12E+05 | 3.99E-05 | 1.88E-10 |
| 1B-1B6-Fc | 8.44E+04 | 6.96E-04 | 8.25E-09 | 5.07E+04 | 3.47E-04 | 6.85E-09 |
| 1B-1A8-Fc | 1.73E+05 | 1.12E-04 | 6.48E-10 | 1.30E+05 | 8.80E-05 | 6.77E-10 |
| 3B-1C7-Fc | 1.76E+05 | 5.56E-05 | 3.17E-10 | 2.89E+05 | 7.35E-05 | 2.54E-10 |

Example 4. Binding of Anti-Human c-Met $V_H$H-Fc Chimeric Antibodies to Natural Cell Lines Expressing c-Met

4.1 Flow cytometry assays for binding of anti-human c-Met $V_H$H-Fc chimeric antibodies to cells

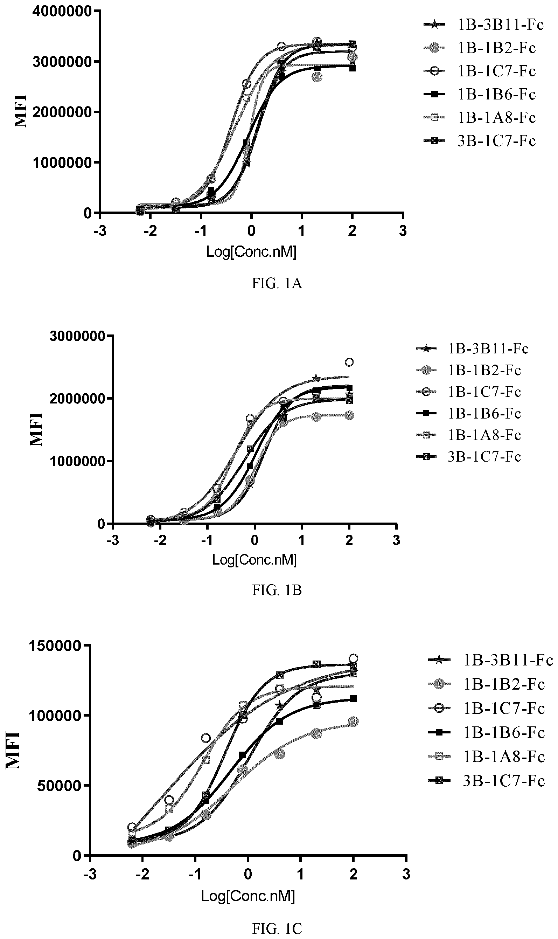

The binding of the anti-human c-Met $V_H$H-Fc chimeric antibody to target cells with different c-Met expression levels was detected by flow cytometry. The H1993 cells (Beina Bio, product catalog No. BNCC342186) are human lung adenocarcinoma cell lines with a high c-Met expression level, the MKN45 cells (Nanjing Cobioer Biosciences Co., Ltd., product catalog No. CBP60541) are human gastric cancer cell lines with a medium c-Met expression level, and the KP4 cells (Nanjing Cobioer Biosciences Co., Ltd., product catalog No. CBP60541) are human pancreatic cancer cell lines with a low c-Met expression level. After $2 \times 10^5$ target cells were incubated with a serially diluted anti-human c-Met $V_H$H-$F_C$ chimeric antibodies (serially diluted 5-fold from the initial concentration of 100 nM to 7 concentrations) on ice for 1 h, the cells were washed. A PE-labeled anti-human IgG Fc antibody (Jackson Immuno Research, product catalog No. 109-116-170) was added, and the mixture was incubated on ice for 0.5 h. The cells were washed and then tested using a flow cytometer (Thermo Fisher Scientific Inc., Attune NXT). The results are shown in FIGs. 1A-1C and Table 2. 1B-1B2-Fc, 1B-3B11-Fc, 1B-1C7-Fc, 1B-1B6-Fc, 1B-1A8-Fc, and 3B-1C7-Fc all have a relatively high binding ability for target cells with different c-Met expression levels.

Table 2. Binding of anti-human c-Met $V_H$H-$F_C$ chimeric antibodies to target cells

| $V_H$H-Fc Chimeric antibodies | H1993 cell | MKN45 cell | KP4 cell |
|---|---|---|---|
| | $EC_{50}$ (nM) | $EC_{50}$ (nM) | $EC_{50}$ (nM) |
| 1B-3B11-Fc | 1.333 | 1.615 | 1.049 |
| 1B-1B2-Fc | 0.910 | 1.034 | 0.526 |
| 1B-1C7-Fc | 0.407 | 0.421 | 0.019 |
| 1B-1B6-Fc | 0.854 | 1.056 | 0.471 |
| 1B-1A8-Fc | 0.477 | 0.345 | 0.143 |
| 3B-1C7-Fc | 1.352 | 0.592 | 0.375 |

4.2 Flow cytometry assays for binding of anti-human c-Met $V_H$H-Fc chimeric antibodies to cells expressing macaque c-Met

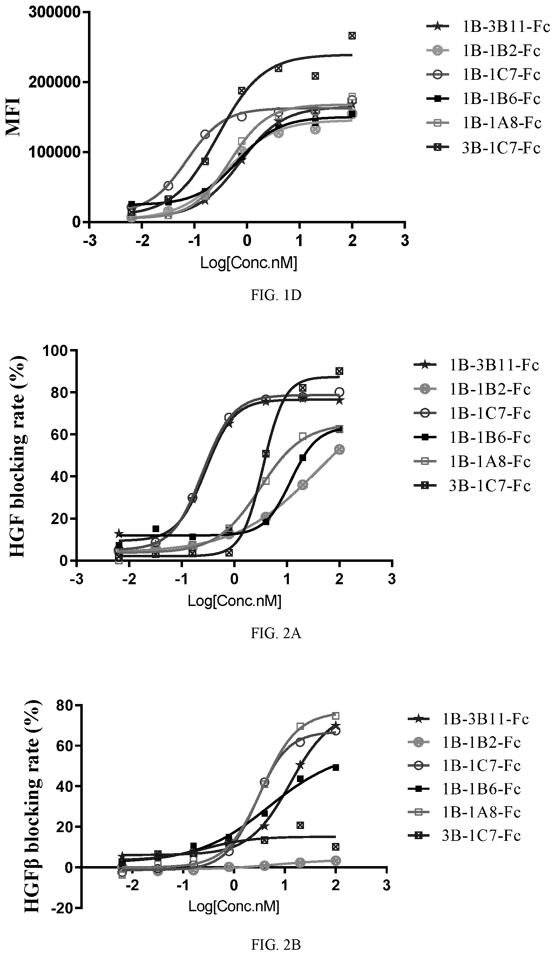

The binding of the anti-human c-Met $V_H$H-Fc chimeric antibody to macaque epithelial cell 4MBr-5 (Shanghai Qingqi Biotechnology Development Co., Ltd., product catalog No. BFN60807362) was detected by flow cytometry by reference to the method of 4.1. The results are shown in FIG. 1D and Table 3. 1B-1B2-Fc, 1B-3B11-Fc, 1B-1C7-Fc, 1B-1B6-Fc, 1B-1A8-Fc, and 3B-1C7-Fc can all effectively target and bind to the macaque epithelial cell 4MBr-5 expressing c-Met.

Table 3. Binding of anti-human c-Met $V_H$H-$F_C$ chimeric antibodies to macaque epithelial cells

| $V_H$H-Fc Chimeric antibodies | 4MBr-5 cell $EC_{50}$ (nM) |
|---|---|
| 1B-3B11-Fc | 0.736 |
| 1B-1B2-Fc | 0.418 |
| 1B-1C7-Fc | 0.071 |
| 1B-1B6-Fc | 0.682 |
| 1B-1A8-Fc | 0.471 |
| 3B-1C7-Fc | 0.284 |

Example 5. Blocking of Receptor/Ligand Binding by Anti-Human c-Met $V_H$H-Fc Chimeric Antibodies and Antibody Epitope Competition Analysis

5.1 Blocking of binding of HGF and HGFβ to c-Met receptor by anti-human c-Met $V_H$H-Fc chimeric antibodies

Whether the anti-human c-Met $V_H$H-Fc chimeric antibody could block the binding of hepatocyte growth factor (HGF) to the c-Met receptor was analyzed by a competitive ELISA method. Specifically, 2 μg/mL human c-Met-His protein (SinoBiological, product catalog No. 10692-H08H) was allowed to coat a high-adsorption microplate, and then the plate was blocked with a 3% (w/v) BSA blocking buffer, washed, then 80 ng/mL biotin-conjugated HGF (SinoBiological, product catalog No. 10463-HNAS) and the anti-human c-Met $V_H$H-Fc chimeric antibodies (serially diluted 5-fold from the initial concentration of 100 nM to 7 concentrations) were added, and incubated at 25°C for 2 h. After the plate was washed, an HRP-conjugated streptavidin (eBioscience, product catalog No. 18-4100-51) was added. The plate was incubated at 25°C for 1 h and then washed 5 times, and a TMB substrate solution (TIANGEN, product catalog No. PA107) was added at 100 μL/well for color development. The light absorption signal values at the wavelength of 450 nm and the reference wavelength of 650 nm were measured. A curve (FIG. 2A) was fit, and the $IC_{50}$ values (Table 4) were calculated.

The same method was used to analyze whether the anti-human c-Met $V_H$H-$F_C$ chimeric antibody could block the binding of the β chain of HGF (HGFβ, NP_000592.3, Val495-Ser728) to the c-Met receptor. The HGFβ has the amino acid sequence set forth in SEQ ID NO: 35. A curve (FIG. 2B) was fit based on the results, and the $IC_{50}$ values were calculated (Table 4).

From the results, 1B-3B11-Fc, 1B-1B2-Fc, 1B-1C7-Fc, 1B-1B6-Fc, 1B-1A8-Fc, and 3B-1C7-Fc can all block the binding of HGF to the c-Met receptor; 1B-3B11-Fc, 1B-1C7-Fc, 1B-1B6-Fc, and 1B-1A8-Fc can block the binding of HGFβ to the c-Met receptor, and 1B-1B2-Fc and 3B-1C7-Fc can not block the binding of HGFβ to the c-Met receptor.

Table 4. Blocking of binding of HGF and HGFβ to c-Met receptor by anti-human c-Met $V_H$H-Fc chimeric antibodies

| $V_H$H-$F_C$ Chimeric antibodies | Blocking of binding of HGF to c-Met | | Blocking of binding of HOPP to c-Met | |
|---|---|---|---|---|
| | $IC_{50}$ (nM) | Maximum blocking rate | $IC_{50}$ (nM) | Maximum blocking rate |
| 1B-3B11-Fc | 0.29 | 76% | 12.52 | 70% |
| 1B-1B2-Fc | 40.90 | 53% | No blocking | / |
| 1B-1C7-Fc | 3.52 | 80% | 2.76 | 67% |
| 1B-1B6-Fc | 11.71 | 62% | 4.45 | 49% |
| 1B-1A8-Fc | 3.14 | 63% | 3.56 | 75% |
| 3B-1C7-Fc | 3.52 | 90% | No blocking | / |

5.2 Investigation of competitive relationships between different clones of anti-human c-Met $V_H$H-$F_C$ by surface plasmon resonance

Epitope competition relationship analysis was performed using a biomolecular interaction system (Fortebio, product catalog No. Octet RED96). An Anti-Penta-HIS (HIS1K) sensor (Fortebio, product catalog No. 18-5120) was used, and the c-Met-His protein (SinoBiological, product catalog No. 10692-H08H) was diluted to about 5 μg/mL using a running buffer. The sensor was immersed in the diluted antigen sample. The immobilization height was controlled to about 1 nm by adjusting the binding time. Then the antigen sample interacted with a first antibody A and a second antibody B in sequence, and the B antibody binding signal was detected to determine whether the two antibodies recognize the same epitope. The results are shown in Table 5-1 and Table 5-2. The determination criteria were: a value > 60% indicated that the 2 antibodies were not competing at all; a value between 20% and 60% indicated a partial competition of the 2 antibodies (there may be a crossover at the epitopes); a value < 20% indicated a complete competition of the 2 antibodies. If the self-reaction signal (gray part) < 20%, the data was determined effective.

As can be seen from the data in Table 5-1, the two antibodies 1B-3B11-Fc and 1B-1A8-Fc competed completely; 1B-3B11-Fc and 1B-1A8-Fc had no obvious competitive relationship with the other three candidate antibodies and belonged to different epitopes respectively. Thus, the antibodies 1B-3B11-Fc and 1B-1B2-Fc, 1B-3B11-Fc and 1B-1C7-Fc, 1B-3B11-Fc and 1B-1B6-Fc, 1B-1A8-Fc and 1B-1B2-Fc, 1B-1A8-Fc and 1B-1C7-Fc, 1B-1A8-Fc and 1B-1B6-Fc, 1B-1B2-Fc and 1B-1C7-Fc, 1B-1B2-Fc and 1B-1B6-Fc, and 1B-1C7-Fc and 1B-1B6-Fc can simultaneously bind to different epitopes of the c-Met antigen.

As can be seen from the data in Table 5-2, the two antibodies 3B-1C7-Fc and 1B-1B2-Fc competed completely. Thus, by combining the data from Table 5-1, it can be seen that: the antibodies 1B-1A8-Fc and 3B-1C7-Fc, 3B-1C7-Fc and 1B-1C7-Fc, and 3B-1C7-Fc and 1B-1B6-Fc can simultaneously bind to different epitopes of the c-Met antigen.

Table 5-1. Epitope difference analysis by surface plasmon resonance

| AB# | 1B-3B11-Fc | 1B-1B2-Fc | 1B-1C7-Fc | 1B-1B6-Fc | 1B-1A8-Fc |
|---|---|---|---|---|---|
| **1B-3B11-Fc** | 9.83% | 132.28% | 118.99% | 126.72% | 8.16% |
| **1B-1B2-Fc** | 120.47% | 7.44% | 120.97% | 122.01% | 117.16% |
| **1B-1C7-Fc** | 91.78% | 87.01% | 0.00% | 97.85% | 91.06% |
| **1B-1B6-Fc** | 125.54% | 119.93% | 118.44% | 13.35% | 125.90% |
| **1B-1A8-Fc** | 20.31% | 122.78% | 116.44% | 122.38% | 17.76% |

Table 5-2. Epitope difference analysis by surface plasmon resonance

| AB# | 1B-3B11-Fc | 1B-1B2-Fc | 1B-1C7-Fc | 1B-1B6-Fc | 3B-1C7-Fc |
|---|---|---|---|---|---|
| **1B-3B11-Fc** | 8.85% | 100.96% | 94.39% | 136.09% | 135.70% |
| **1B-1B2-Fc** | 126.48% | 0.12% | 91.08% | 120.10% | 7.11% |
| **1B-1C7-Fc** | 126.48% | 95.31% | 7.51% | 126.45% | 122.76% |
| **1B-1B6-Fc** | 133.73% | 122.60% | 121.83% | 5.41% | 117.22% |
| **3B-1C7-Fc** | 137.10% | 12.03% | 141.34% | 161.78% | 13.40% |

Example 6. Inhibition of c-Met Phosphorylation in H441 Cells by Anti-Human c-Met $V_HH$-Fc Chimeric Antibodies

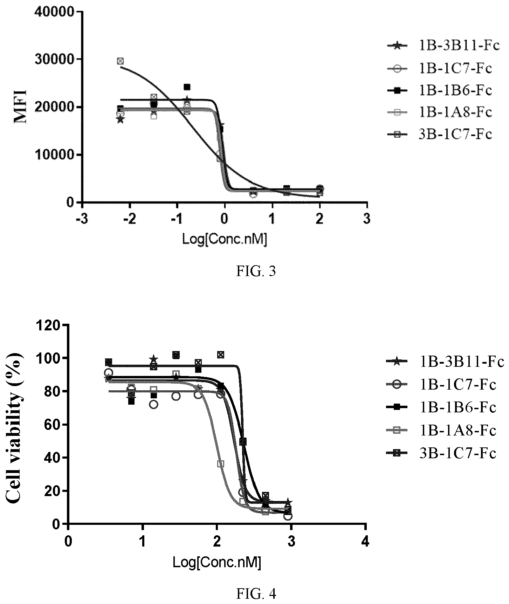

The inhibitory effect of the antibody on c-Met phosphorylation caused by HGF stimulation in H441 cells (Beina Bio, product catalog No. BNCC292357) was detected using the Phospho-c-Met (Tyr1234/1235) AlphaLISA SureFire Ultra Assay Kit (PerkinElmer, product catalog No. ALSU-PC-MET-A500). According to the supplier's instructions, H441 cells were taken and inoculated in a 96-well plate at 100 $\mu$L/well and 10000 cells/well. After 6-16 h of incubation, the cells were starved overnight in a serum-free culture medium. The supernatant was discarded, the anti-human c-Met $V_HH$-Fc chimeric antibodies (serially diluted 10-fold from the initial concentration of 1000 nM to 7 concentrations) were added at 45 $\mu$L/well, the mixture was incubated for 30 min, and 5 $\mu$L of HGF was added for stimulation for 15 min. The supernatant was discarded, and 100 $\mu$L of a freshly prepared 1× lysis buffer was added. After 10 min of incubation, the plate was read using the AlphaLISA module. The results are shown in FIG. 3 and Table 6. 1B-1B2-Fc, 1B-3B11-Fc, 1B-1C7-Fc, 1B-1B6-Fc, 1B-1A8-Fc, and 3B-1C7-Fc can all effectively inhibit the c-Met downstream phosphorylation of in H441 cells caused by HGF stimulation.

Table 6. Inhibition of c-Met phosphorylation in H441 cells by anti-human c-Met $V_HH$-Fc chimeric antibodies

| $V_HH$-$F_C$ | Phosphorylation inhibition | |
|---|---|---|
| Chimeric antibodies | $IC_{50}$ (nM) | Maximum inhibition rate |
| 1B-3B11-Fc | 0.95 | 91% |
| 1B-1C7-Fc | 0.79 | 90% |
| 1B-1B6-Fc | 0.86 | 88% |
| 1B-1A8-Fc | 0.85 | 90% |
| 3B-1C7-Fc | 0.21 | 94% |

Example 7. Inhibition of Proliferation of KP4 Cells by Anti-Human c-Met $V_HH$-$F_C$ Chimeric Antibodies

The inhibitory effect of the antibody on the proliferation of KP4 cells was detected using the Cell Counting Kit 8 (DOJINDO Laboratories, Lot.SH797) method. Specifically, KP4 cells were added to a 96-well cell culture plate at 1000 cells/well and incubated in a 5% $CO_2$ carbon dioxide cell incubator at 37°C. After the cells were adhered to the wall, the anti-human c-Met $V_HH$-Fc chimeric antibodies (serially diluted 2-fold from the initial concentration of 900 nM to 7 concentrations) were added to the cells. After 120 h of incubation, 10 $\mu$L of CCK-8 solution was added to each well. After 1 h of incubation, the absorbance at 450 nm was measured using a microplate reader. The results are shown in FIG. 4 and Table 7. 1B-3B11-Fc, 1B-1C7-Fc, 1B-1B6-Fc, 1B-1A8-Fc, and 3B-1C7-Fc can all effectively inhibit the proliferation of KP4 cells.

The cell viability calculation formula is as follows:

$$\text{Cell viability} = [(As - Ab)/(Ac - Ab)] \times 100\%,$$

As = experimental well absorbance (absorbance of wells containing cells, culture medium, CCK-8, and the test antibodies),

Ab = blank well absorbance (absorbance of wells containing culture medium and CCK-8),

Ac = control well absorbance (absorbance of wells containing cells, culture medium, and CCK-8);

$$\text{Proliferation inhibition rate} = 100\% - \text{cell viability.}$$

Table 7. Inhibition of Proliferation of KP4 cells by Anti-Human c-Met $V_HH$-Fc Chimeric Antibodies

| $V_HH$-Fc | KP4 cell proliferation inhibition | |
|---|---|---|
| Chimeric antibodies | $IC_{50}$ (nM) | Maximum inhibition rate |
| 1B-3B11-Fc | 171.5 | 87% |
| 1B-1C7-Fc | 183.3 | 95% |

(continued)

| $V_HH$-Fc | KP4 cell proliferation inhibition | |
|---|---|---|
| Chimeric antibodies | $IC_{50}$ (nM) | Maximum inhibition rate |
| 1B-1B6-Fc | 231.2 | 93% |
| 1B-1A8-Fc | 99.8 | 91% |
| 3B-1C7-Fc | 223.2 | 91% |

**Claims**

1. An isolated antibody or an antigen-binding fragment thereof, wherein the antibody or the antigen-binding fragment thereof binds to c-Met and comprises at least one single variable domain, wherein the single variable domain comprises the following complementarity determining regions:

   (a) CDR1 comprising the amino acid sequence set forth in SEQ ID NO: 11, 8, 5, 14, 17, or 20;
   (b) CDR2 comprising the amino acid sequence set forth in SEQ ID NO: 12, 9, 6, 15, 18, or 21; and
   (c) CDR3 comprising the amino acid sequence set forth in SEQ ID NO: 13, 10, 7, 16, 19, or 22.

2. The isolated antibody or the antigen-binding fragment thereof according to claim 1, wherein the single variable domain comprises:

   (a) CDR1 comprising the amino acid sequence set forth in SEQ ID NO: 11, CDR2 comprising the amino acid sequence set forth in SEQ ID NO: 12, and CDR3 comprising the amino acid sequence set forth in SEQ ID NO: 13;
   (b) CDR1 comprising the amino acid sequence set forth in SEQ ID NO: 8, CDR2 comprising the amino acid sequence set forth in SEQ ID NO: 9, and CDR3 comprising the amino acid sequence set forth in SEQ ID NO: 10;
   (c) CDR1 comprising the amino acid sequence set forth in SEQ ID NO: 5, CDR2 comprising the amino acid sequence set forth in SEQ ID NO: 6, and CDR3 comprising the amino acid sequence set forth in SEQ ID NO: 7;
   (d) CDR1 comprising the amino acid sequence set forth in SEQ ID NO: 14, CDR2 comprising the amino acid sequence set forth in SEQ ID NO: 15, and CDR3 comprising the amino acid sequence set forth in SEQ ID NO: 16;
   (e) CDR1 comprising the amino acid sequence set forth in SEQ ID NO: 17, CDR2 comprising the amino acid sequence set forth in SEQ ID NO: 18, and CDR3 comprising the amino acid sequence set forth in SEQ ID NO: 19; or
   (f) CDR1 comprising the amino acid sequence set forth in SEQ ID NO: 20, CDR2 comprising the amino acid sequence set forth in SEQ ID NO: 21, and CDR3 comprising the amino acid sequence set forth in SEQ ID NO: 22.

3. The isolated antibody or the antigen-binding fragment thereof according to claim 2, wherein the single variable domain comprises an amino acid sequence having at least 85% identity to the amino acid sequence set forth in SEQ ID NO: 24, 26, 28, 30, 32, or 34, or
   the single variable domain comprises the amino acid sequence set forth in SEQ ID NO: 24, 26, 28, 30, 32, or 34.

4. The isolated antibody or the antigen-binding fragment thereof according to any one of claims 1 to 3, wherein the single variable domain is a $V_HH$; optionally, the antibody or the antigen-binding fragment thereof is chimeric or humanized.

5. The isolated antibody or the antigen-binding fragment thereof according to any one of claims 1 to 4, wherein the antibody or the antigen-binding fragment thereof comprises an Fc of human IgG, preferably an Fc of human IgG1, IgG2, IgG3, or IgG4; optionally, the antibody or the antigen-binding fragment thereof comprises the amino acid sequence set forth in SEQ ID NO: 37, 39, 41, 43, 45, or 47.

6. The isolated antibody or the antigen-binding fragment thereof according to any one of claims 1 to 5, wherein the antibody or the antigen-binding fragment thereof may comprise one or several of the following properties and characteristics, alone or in combination:

   (a) binding to human c-Met or/and monkey c-Met;
   (b) blocking the binding of HGF or a β chain thereof to c-Met;
   (c) inhibiting c-Met phosphorylation caused by HGF stimulation;

(d) exerting the ADCC effect on tumor cells.

7. A fusion protein comprising the antibody or the antigen-binding fragment thereof according to any one of claims 1 to 6.

8. A pharmaceutical composition comprising the antibody or the antigen-binding fragment thereof according to any one of claims 1 to 6, or the fusion protein according to claim 7, and a pharmaceutically acceptable carrier.

9. An isolated nucleic acid encoding the antibody or the antigen-binding fragment thereof according to any one of claims 1 to 6 or the fusion protein according to claim 7.

10. A vector comprising the isolated nucleic acid according to claim 9.

11. A host cell comprising the nucleic acid according to claim 9 or the vector according to claim 10.

12. A method for preparing the isolated antibody or the antigen-binding fragment thereof according to any one of claims 1 to 6, comprising: culturing the host cell according to claim 11 and isolating the antibody or the antigen-binding fragment thereof expressed, wherein the vector is an expression vector.

13. Use of the isolated antibody or the antigen-binding fragment thereof according to any one of claims 1-6, the fusion protein according to claim 7, or the pharmaceutical composition according to claim 8 for preparing a medicament for treating a subject having a tumor, wherein preferably, the tumor is brain cancer, head and neck cancer, lung cancer, esophageal cancer, pharyngeal cancer, nasal cancer, tongue cancer, oral cancer, thyroid cancer, skin cancer, bone cancer, soft tissue sarcoma, myeloma, gastric cancer, gastroesophageal junction adenocarcinoma, breast cancer, liver cancer, kidney cancer, pancreatic cancer, spleen cancer, lymphoma, thymus cancer, bladder cancer, vaginal cancer, testicular cancer, uterine cancer, cervical cancer, colorectal cancer, anal cancer, papillary carcinoma, prostate cancer, ovarian cancer, epithelial cell cancer, or hematologic tumor.

14. A method for detecting or measuring c-Met in a sample, comprising contacting the sample with the antibody or the antigen-binding fragment thereof according to any one of claims 1 to 6, and detecting or measuring a binding complex.

15. A method for inhibiting, reducing or blocking c-Met signaling in a cell, comprising administering to the cell an effective amount of the antibody or the antigen-binding fragment thereof according to any one of claims 1 to 6, the fusion protein according to claim 7, or the pharmaceutical composition according to claim 8.

16. A method for inhibiting tumor cell growth or killing a tumor cell, comprising administering to the tumor cell an effective amount of the antibody or the antigen-binding fragment thereof according to any one of claims 1 to 6, the fusion protein according to claim 7, or the pharmaceutical composition according to claim 8.

17. A method for treating a subject having a tumor, comprising administering to the subject a therapeutically effective amount of the antibody or the antigen-binding fragment thereof according to any one of claims 1 to 6, the fusion protein according to claim 7, or the pharmaceutical composition according to claim 8.

18. The method according to claim 16 or 17, wherein the tumor is brain cancer, head and neck cancer, lung cancer, esophageal cancer, pharyngeal cancer, nasal cancer, tongue cancer, oral cancer, thyroid cancer, skin cancer, bone cancer, soft tissue sarcoma, myeloma, gastric cancer, gastroesophageal junction adenocarcinoma, breast cancer, liver cancer, kidney cancer, pancreatic cancer, spleen cancer, lymphoma, thymus cancer, bladder cancer, vaginal cancer, testicular cancer, uterine cancer, cervical cancer, colorectal cancer, anal cancer, papillary carcinoma, prostate cancer, ovarian cancer, epithelial cell cancer, or hematologic tumor.

FIG. 1A

FIG. 1B

FIG. 1C

FIG. 1D

FIG. 2A

FIG. 2B

FIG. 3

FIG. 4

## INTERNATIONAL SEARCH REPORT

| International application No. |
| --- |
| **PCT/CN2022/129875** |

| A. | CLASSIFICATION OF SUBJECT MATTER |
| --- | --- |

C07K 16/28(2006.01)i; C12N 15/13(2006.01)i; A61K 39/395(2006.01)i; A61P 35/00(2006.01)i

According to International Patent Classification (IPC) or to both national classification and IPC

| B. | FIELDS SEARCHED |
| --- | --- |

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

VEN, CNABS, PubMed, ISI Web of Science, VCN, CNKI, 万方, WANFANG, 百度学术, BAIDU SCHOLAR, 读秀, DUXIU, STN, GenBank, 中国专利生物序列检索系统, Chinese Patent Biological Sequence Retrieval System: c-Met, 间质表皮转化因子, c-Mesenchymal-epithelial transition factor, 纳米抗体, 单域抗体, VHH, nanobody, 癌, 肿瘤, cancer, carcinoma, tumour, tumor, SEQ ID NOs: 6-22.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 103889451 A (ABLYNX NV) 25 June 2014 (2014-06-25)<br>entire document | 1-18 |
| A | US 2005054019 A1 (MICHAUD, N. R. et al.) 10 March 2005 (2005-03-10)<br>entire document | 1-18 |
| A | 季慧凝 等 (JI, Huining et al.). "纳米抗体在癌症治疗方面研究进展 (Research Progress of Nanobody in Cancer Therapy)"<br>生命的化学 (Chemistry of Life), Vol. 37, No. 04, 31 December 2017 (2017-12-31),<br>pages 533-542 | 1-18 |
| A | CN 103183738 A (NOVARTIS AG) 03 July 2013 (2013-07-03)<br>entire document | 1-18 |
| A | CN 106267191 A (SHANXI NAAN BIOTECHNOLOGY CO., LTD.) 04 January 2017<br>(2017-01-04)<br>entire document | 1-18 |

| ✓ Further documents are listed in the continuation of Box C. | ✓ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **30 December 2022** | **11 January 2023** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/129875** |

**C.** **DOCUMENTS CONSIDERED TO BE RELEVANT**

| Category* | Citation of document, with indication, where appropriate, of the relevant passages | Relevant to claim No. |
| --- | --- | --- |
| A | FUJITA, R. et al. "A novel non-agonist c-Met antibody drug conjugate with superior potency over a c-Met tyrosine kinase inhibitor in c-Met amplified and non-amplified cancers" *CANCER BIOLOGY & THERAPY*, Vol. 21, No. 6, 31 December 2020 (2020-12-31), pages 549-559 | 1-18 |
| A | LU, R. M. et al. "Single chain anti-c-Met antibody conjugated nanoparticles for in vivo tumor-targeted imaging and drug delivery" *Biomaterials*, Vol. 32, 07 February 2011 (2011-02-07), pages 3265-3274 | 1-18 |

Form PCT/ISA/210 (second sheet) (January 2015)

**INTERNATIONAL SEARCH REPORT**

International application No.

**PCT/CN2022/129875**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

a. ☑ forming part of the international application as filed:

☑ in the form of an Annex C/ST.25 text file.

☐ on paper or in the form of an image file.

b. ☐ furnished together with the international application under PCT Rule 13*ter*.1(a) for the purposes of international search only in the form of an Annex C/ST.25 text file.

c. ☐ furnished subsequent to the international filing date for the purposes of international search only:

☐ in the form of an Annex C/ST.25 text file (Rule 13*ter*.1(a)).

☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

[1]   The actually submitted sequence table is an XML file of the ST.26 standard.

## INTERNATIONAL SEARCH REPORT

International application No.

**PCT/CN2022/129875**

| Box No. II    Observations where certain claims were found unsearchable (Continuation of item 2 of first sheet) |
| --- |

This international search report has not been established in respect of certain claims under Article 17(2)(a) for the following reasons:

1. ☑ Claims Nos.: **15-18**
   because they relate to subject matter not required to be searched by this Authority, namely:

   [1]   Claim 15 relates to a method for inhibiting, reducing or blocking c-Met signaling in a cell, and claims 16-18 relate to a method for inhibiting tumor growth or killing a tumor cell and a method for treating a subject suffering from a tumor, belonging to disease treatment methods as defined in PCT Rule 39.1(iv). The search was made on the basis of a corresponding pharmaceutical use of an antibody or an antigen-binding fragment and a pharmaceutical composition thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

International application No.

**PCT/CN2022/129875**

| Patent document cited in search report | | | Publication date (day/month/year) | Patent family member(s) | | | Publication date (day/month/year) |
|---|---|---|---|---|---|---|---|
| CN | 103889451 | A | 25 June 2014 | AU | 2013201446 | A1 | 02 May 2013 |
| | | | | CN | 106046161 | A | 26 October 2016 |
| | | | | EP | 2747783 | A2 | 02 July 2014 |
| | | | | CA | 2850261 | A1 | 04 April 2013 |
| | | | | IN | 3147CHENP2014 | A | 12 December 2014 |
| | | | | AU | 2013201446 | B9 | 31 March 2016 |
| | | | | WO | 2013045707 | A2 | 04 April 2013 |
| | | | | JP | 2015501135 | A | 15 January 2015 |
| US | 2005054019 | A1 | 10 March 2005 | HN | 2004000285 | A | 27 April 2006 |
| | | | | JP | 2007501013 | A | 25 January 2007 |
| | | | | CA | 2534563 | A1 | 24 February 2005 |
| | | | | NL | 1026776 | A1 | 07 February 2005 |
| | | | | UY | 28453 | A1 | 28 February 2005 |
| | | | | TW | 200523269 | A | 16 July 2005 |
| | | | | US | 2014086914 | A1 | 27 March 2014 |
| | | | | US | 2012321614 | A1 | 20 December 2012 |
| | | | | AR | 047717 | A1 | 15 February 2006 |
| | | | | BR | PI0413272 | A | 10 October 2006 |
| | | | | PA | 8608401 | A1 | 03 March 2005 |
| | | | | GB | 0417384 | D0 | 08 September 2004 |
| | | | | US | 2010040629 | A1 | 18 February 2010 |
| | | | | GT | 200400149 | A | 22 February 2005 |
| | | | | PE | 20050727 | A1 | 01 October 2005 |
| | | | | WO | 2005016382 | A1 | 24 February 2005 |
| | | | | EP | 1660127 | A1 | 31 May 2006 |
| CN | 103183738 | A | 03 July 2013 | MX | 2008012485 | A | 10 October 2008 |
| | | | | KR | 20080113218 | A | 29 December 2008 |
| | | | | CL | 2007000851 | A1 | 14 March 2008 |
| | | | | US | 2009175860 | A1 | 09 July 2009 |
| | | | | CA | 2646048 | A1 | 08 November 2007 |
| | | | | TW | 200815470 | A | 01 April 2008 |
| | | | | RU | 2008142833 | A | 10 May 2010 |
| | | | | AR | 060241 | A1 | 04 June 2008 |
| | | | | CN | 101415730 | A | 22 April 2009 |
| | | | | WO | 2007126799 | A2 | 08 November 2007 |
| | | | | EP | 2004693 | A2 | 24 December 2008 |
| | | | | JP | 2009532026 | A | 10 September 2009 |
| | | | | ES | 2386738 | T3 | 28 August 2012 |
| | | | | AU | 2007245181 | A1 | 08 November 2007 |
| | | | | PE | 20080352 | A1 | 12 June 2008 |
| | | | | BR | PI0709917 | A2 | 05 July 2011 |
| CN | 106267191 | A | 04 January 2017 | US | 2019184012 | A1 | 20 June 2019 |
| | | | | WO | 2018041269 | A1 | 08 March 2018 |

Form PCT/ISA/210 (patent family annex) (January 2015)

## REFERENCES CITED IN THE DESCRIPTION

**Non-patent literature cited in the description**

- **ELVIN A. KABAT et al.** Sequences of Proteins of Immunological Interest. Public Health Service, National Institutes of Health, 1991
- **CYRUS CHOTHIA et al.** Canonical Structures for the Hypervariable Regions of Immunoglobulins. *J. Mol. Biol.,* 1987, vol. 196, 901-917
- **KABAT et al.** Sequences of Proteins of Immunological Interest. Public Health Service, National Institutes of Health, 1991

- Remington's Pharmaceutical Sciences. Mack Printing Company, 1990, 1289-1329
- **E. MEYERS ; W MILLER.** *Comput. Appl. Biosci.,* 1988, vol. 4, 11-17
- **NEEDLEMAN ; WUNSCH.** *J. Mol. Biol.,* 1970, 484-453